(19) Europäisches Patentamt / European Patent Office / Office européen des brevets

(11) **EP 3 900 623 B1**

(12) **EUROPEAN PATENT SPECIFICATION**

(45) Date of publication and mention
of the grant of the patent:
**30.07.2025   Bulletin 2025/31**

(21) Application number: **20170650.4**

(22) Date of filing: **21.04.2020**

(51) International Patent Classification (IPC):
*A61B 5/30* (2021.01)     *A61B 5/392* (2021.01)

(52) Cooperative Patent Classification (CPC):
**A61B 5/7264; A61B 5/30; A61B 5/392;
A61B 5/7282**

(54) **MEDICAL DIAGNOSTIC SYSTEM COMPRISING A PORTABLE MEASURING DEVICE FOR DETECTING AND IDENTIFYING PHYSIOLOGICAL CHANGES IN GASTROINTESTINAL ACTIVITY**

MEDIZINISCHES DIAGNOSESYSTEM MIT TRAGBARER MESSVORRICHTUNG ZUR DETEKTION UND IDENTIFIZIERUNG PHYSIOLOGISCHER VERÄNDERUNGEN DER GASTROINTESTINALEN AKTIVITÄT

SYSTÈME DE DIAGNOSTIC MÉDICAL DE DÉTECTION ET D'IDENTIFICATION DES CHANGEMENTS PHYSIOLOGIQUES DANS UNE ACTIVITÉ GASTRO-INTESTINALE COMPRENANT UN DISPOSITIF DE MESURE PORTABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.10.2021   Bulletin 2021/43**

(73) Proprietor: **MDE Kft.**
**1062 Budapest (HU)**

(72) Inventors:
• **GROSZ, Tamás**
**1089 Budapest (HU)**
• **GROSZ, György**
**8230 Balatonfüred (HU)**
• **SÜLE, Miklós**
**8200 Veszprém (HU)**
• **GÁSPÁR, Habil, Róbert**
**6726 Szeged (HU)**
• **SZÜCS, Kálmán**
**6722 Szeged (HU)**

(74) Representative: **Danubia Patent & Law Office LLC**
**Bajcsy-Zsilinszky út 16**
**1051 Budapest (HU)**

(56) References cited:
**EP-A1- 0 687 442     US-A- 5 795 304**

• **PIETRASZEK S ET AL: "The simultaneous recording and analysis both EGG and HRV signals", PROCEEDINGS OF THE 31ST ANNUAL INTERNATIONAL CONFERENCE OF THE IEEE ENGINEERING IN MEDICINE AND BIOLOGY SOCIETY: ENGINEERING THE FUTURE OF BIOMEDICINE, EMBC 2009, IEEE, 3 September 2009 (2009-09-03), pages 396 - 399, XP031881629, ISBN: 978-1-4244-3296-7, DOI: 10.1109/IEMBS.2009.5333455**
• **S. HADDAB ET AL: "Microcontroller - Based System for Electrogastrography Monitoring Through Wireless Transmission", MEASUREMENT SCIENCE REVIEW, vol. 9, no. 5, 1 January 2009 (2009-01-01), XP055491503, DOI: 10.2478/v10048-009-0022-6**
• **J. YIN ET AL: "Inhibitory effects of stress on postprandial gastric myoelectrical activity and vagal tone in healthy subjects", NEUROGASTROENTEROLOGY AND MOTILITY, vol. 16, no. 6, 1 December 2004 (2004-12-01), GB, pages 737 - 744, XP055733958, ISSN: 1350-1925, DOI: 10.1111/j.1365-2982.2004.00544.x**

**Description**

The invention relates to a medical diagnostic system for measuring and transmitting or recording physiological signals emanating from human or animal body. Based on the analysis of the measured signals, the system according to the invention enables the detection of changes in visceral activity and determination of the extent thereof and thus enables detection of physiological conditions associated with the visceral activity, as well as diagnosis of anomalies and pathological abnormalities associated with the visceral activity, particularly of gastro-esophageal reflux disease and anxiety.

There is a long-felt need for reliable and minimally invasive diagnosis of gastro-esophageal reflux disease and reliable detection of psychological stress or anxiety experienced by patients. Numerous attempts have been made to diagnose gastro-esophageal reflux disease by electrogastrography (EGG), i.e. by analyzing electric activity of the stomach, or more precisely by the analysis of the so-called myoelectric signals emanating from the smooth muscles of the stomach. Such an attempt is disclosed in the publication of T. Banach et al. entitled "Myoelectric activity of the stomach and esophageal pH changes in reflux disease" (Folia Med Cracov. 2001;42(1-2):53-61.), reporting a significant relation between myoelectric activity of the stomach and the presence of reflux disease, however, no parameters are disclosed that could be used for diagnosing the disease without preliminary knowledge thereof.

The publication of Francisco Miguel Vargas-Luna et al entitled "Heart Rate Variability and Gastric Electrical Response to a Cold Pressor Task in Youth with Functional Dyspepsia" (Digestive Diseases and Sciences, Volume 65 (4), 2020) discloses a system for measuring and analyzing electric signal emanating from the muscles of the stomach and the heart. The system comprises two electrodes arranged on the abdomen for simultaneously measuring electric signals emanating from the heart and the stomach. These signals are digitized together and subsequently filtered and separated by software, then analyzed. The publication discusses the effects of stress on different parameters concerning the operation of said organs, however, the results show only slight changes in some of the analyzed parameters, apparent only with the knowledge of the stress factor, i.e. they do not provide a solution for diagnosing a disease or condition without preliminary knowledge thereof.

The publication of Yun Liu et al. entitled "Psychological stress level detection based on electrodermal activity" (Behavioural Brain Research, Volume 341, 2018) discloses a method for detection of stress based on a single physiological parameter and compares its effectiveness with methods that are based on more than one physiological parameters. According to the results disclosed in the publication, the method using only the electrodermal activity (EDA, also known as galvanic skin response, GSR) may detect the psychological stress experienced by the patient with an accuracy above 80%, however, the analysis of multiple physiological parameters beside EDA, e.g. electrocardiogram (ECG), electromyogram (EMG) and respiration data, is assumed to provide more accurate results.

The publication of S. Pietraszek et al. entitled "The simultaneous recording and analysis both EGG and HRV signals" (PROCEEDINGS OF THE 31ST ANNUAL INTERNATIONAL CONFERENCE OF THE IEEE ENGINEERING IN MEDICINE AND BIOLOGY SOCIETY: ENGINEERING THE FUTURE OF BIOMED-ICINE, EMBC 2009, 20090903 IEEE, pp. 396 - 399) discloses a method for synchronous recording and analyzing both the electrogastrographic signal (EGG) and the heart rate variability signal (HRV). The four channel signal are non-invasively captured by the appropriately placed electrodes on the surface of the stomach. The EGG and electrocardiographic (ECG) signals, recorded simultaneously by means of the same electrodes and an amplifier, are separated by the digital filtration.

The publication of J. Yin et al. titled "Inhibitory effects of stress on postprandial gastric myoelectrical activity and vagal tone in healthy subjects" (NEUROGASTROENTEROLOGY AND MOTILITY, 20041201 BLACKWELL SCIENTIFIC PUBLICATIONS, OXFORD, GB, Vol:16, Nr:6, Pages 737 - 744) discloses a study of gastric myoelectrical activity (GMA) and vagal activity in response to stress. Non-invasive measurement and diagnostic solutions of the prior art are not sufficiently accurate for reliable diagnosis of e.g. gastro-esophageal reflux disease, while there is also a need for increasing the accuracy of solutions for detecting stress or anxiety. Limitations of the prior art solutions originate from deficiencies of the measurement systems and/or deficiencies of the evaluation of the measurement results.

The objective of the invention is to provide a medical diagnostic system with increased accuracy that eliminates the aforementioned deficiencies and that is suitable among others for diagnosing gastro-esophageal reflux disease with increased reliability, and for detection of stress or anxiety with increased accuracy.

A substantial inventive recognition is that both the gastro-esophageal reflux disease and the psychological stress experienced by a patient may be more effectively diagnosed, if the measurements and the data analysis are extended to the analysis of the electric activity of the intestines, i.e. if instead of performing only electrogastrography (EGG) measurements, electrogastrointestinography (EGIG) and ECG measurements are performed simultaneously and with correct phase. A further inventive recognition is that the accuracy of the diagnosis may be further improved if electrodermal activity (GSR) measurements and/or body temperature measurements are also performed and the measurement data are processed without relative delays therebetween (and in the case of EGIG and ECG signals also in a phase correct manner) for further data analysis.

In order to accomplish the above objective, several technical problems had to be solved. First, the measuring system has to be capable of simultaneous and highly accurate measurement of physiological signals, e.g. electric signals emanating from different organs. The accuracy of the diagnostic method requires the measured electric signals not to undergo phase shift relative to each other, i.e. a phase correct measurement is required. After obtaining the sufficiently accurate measurement results, a further problem to be solved is the suitable analysis thereof for obtaining the diagnostic result.

A further recognition is that accuracy of a diagnosis produced on the basis of statistical analysis of ECG and EGIG data may be further improved by the use of body temperature and galvanic skin response (GSR) data, i.e. by adding a body temperature sensor and a GSR sensor to the measuring device. The desired accuracy of the diagnostic method according to the invention further requires removing the electric signals of other organs from the ECG and EGIG electric signals of the investigated organ.

The primary objective is to provide a medical diagnostic system comprising a non-invasive measuring device that is suitable for performing prolonged (preferably several hours, e.g. 24 hours) measurements on a person (or animal) for obtaining simultaneously and in correct phase several different physiological electric signals of biological origins that are in different frequency ranges and for storing said signals separately for analysis to be performed later. A further objective is to minimize the discomfort caused to the patient by wearing the measuring device.

The aforementioned objectives have been achieved by providing a medical diagnostic system according to claim 1. Preferred embodiments of the system are disclosed in claims 2-5.

In what follows preferred exemplary embodiments of the diagnostic system according to the invention, as well as the operation thereof are described in detail with reference to the accompanying drawings, wherein

- Figure 1A is a functional block diagram of a basic variant of the portable measuring device according to the invention;
- Figure 1B is a functional block diagram of a preferred embodiment of the portable measuring device according to the invention;
- Figure 2A is a circuit diagram of a myoelectric measurement unit used in a preferred embodiment of the portable measuring device according to the invention;
- Figure 2B is a circuit diagram of an artificial ground circuit used in a preferred embodiment of the portable measuring device according to the invention;
- Figure 2C is a circuit diagram of a temperature measurement unit used in a preferred embodiment of the portable measuring device according to the invention;
- Figure 2D is a circuit diagram of a GSR measurement unit used in a preferred embodiment of the portable measuring device according to the invention;
- Figure 2E shows a functional block diagram of the medical diagnostic system according to the invention;
- Figure 3 is a diagram showing the arrangement of the medical diagnostic system according to the invention;
- Figure 4 shows the course of clinical test for evaluating stress or anxiety;
- Figure 5 shows a preferred exemplary arrangement of electrodes used for detecting stress or anxiety on a living human subject;
- Figure 6 is a flow-chart showing the course of the second stage of processing used for detecting stress or anxiety;
- Figure 7 is a diagram showing an evaluation of the total stomach power;
- Figure 8 is a diagram showing an evaluation of the total small intestine power;
- Figure 9 is a diagram showing a frequency based evaluation of the total HRV;
- Figure 10 is a diagram showing a time based evaluation (pRR50%) of the total HRV;
- Figure 11 is a diagram showing an evaluation of the total GSR in the complete range;
- Figure 12 is a diagram showing an evaluation of the total TH (°C) in the complete range;
- Figure 13 is a diagram showing the results of verification of the logistic regression by ROC analysis;
- Figure 14 shows an exemplary arrangement of the electrodes on a living human subject;
- Figure 15 shows an exemplary display of the ECG and EGIG measurement data;
- Figure 16 shows an exemplary display of the ECG and EGIG measurement data;
- Figure 17 shows an exemplary graphic display of the HRV data;
- Figure 18 is a graph showing the age distribution of persons participating in the test;
- Figure 19 is a flow chart showing the selection and diagnosis of persons participating in the test;
- Figure 20 is a flow chart illustrating an exemplary variant of the diagnostic method according to the invention;
- Figure 21 illustrates the efficiency of an exemplary variant of the diagnostic method according to the invention in diagnosing gastro-esophageal reflux disease;
- Figure 22 illustrates the efficiency of an exemplary variant of the diagnostic method according to the invention in diagnosing gastro-esophageal reflux disease;
- Figure 23 illustrates the efficiency of an exemplary variant of the diagnostic method according to the invention in diagnosing gastro-esophageal reflux disease;
- Figure 24 illustrates the efficiency of an exemplary variant of the diagnostic method according to the invention in diagnosing gastro-esophageal reflux disease.

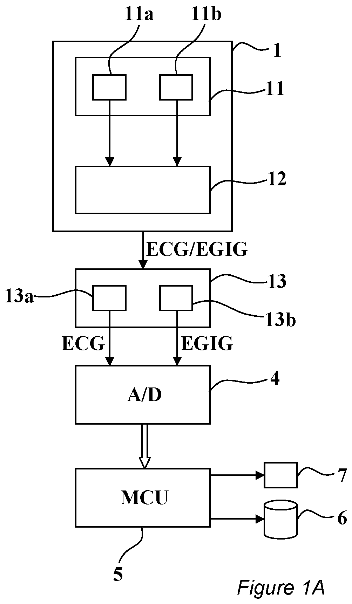

Figure 1A is a functional block diagram of a basic variant of the portable measuring device according to the invention. The portable device, similarly to a Holter device, comprises every necessary component for measuring physiological functions, and storage and/or transmission of the measurement results.

The portable device comprises a measurement unit 1 suitable for measuring physiological electric signals emanating from human or animal muscle tissue. The measurement unit 1 is preferably configured for measuring electric signals emanating from muscle tissue, particularly electric signals of the muscles of the heart and organs of the visceral region, e.g. the stomach, the small intestine and the large intestine. For measuring physiological signals, the measurement unit 1 comprises an electrode pair 11 consisting of two electrodes 11a and 11b. Both electrodes 11a, 11b are configured for the simultaneous detection of at least two physiological signals. This way the electric signals forming the physiological signals, e.g. the EGIG signal and the ECG signal appear simultaneously on the electrodes 11a, 11b and thus they are always in correct phase. The electrodes 11a, 11b are connected to a broadband signal amplifier 12 that produces an amplified EGIG/ECG signal.

The portable measuring device further comprises a simultaneous filter 13 connected to the measurement unit 1 and receiving the output signal of the broadband signal amplifier 12. The simultaneous filter 13 comprises a first filter circuit 13a having first filter characteristics, wherein said first filter circuit 13a separates one of the physiological signals from the output signal of the signal amplifier 12, e.g. it separates the ECG electric signals. The simultaneous filter 13 further comprises a second filter circuit 13b, having a second filter characteristics that are different from the first filter characteristics, wherein said second filter circuit 13b separates a further physiological signal from the output signal of the signal amplifier 12, e.g. it separates the EGIG electric signals. The filter circuits 13a, 13b are tuned so that the phase of the electric signals passed through them are not distorted or distorted only to a negligible extent.

The outputs of the filter circuits 13a, 13b are connected to an input of an A/D converter unit 4, which converts the separated analogue physiological signals to digital signals, then multiplexes said digital signals to its output.

The output of the A/D converter unit 4 is connected to a microcontroller unit 5, or optionally the A/D converter unit 4 may be integrated into the microcontroller unit 5 itself. The function of the microcontroller unit 5 is the conversion of the incoming digital signals to measurement data, handling and organizing the obtained measurement data, selection of an operation mode, and transferring the prepared data to a central computer. These tasks are performed by a firmware program stored in the microcontroller unit 5.

Storage of the digital signals may be performed by a data storage 6 connected to the microcontroller unit 5. Transferring the digital measurement data obtained as a result of the signal processing from the microcontroller unit 5 to a central computer may be performed by a wired or wireless communication interface 7. The data storage 6 may be e.g. a flash drive or an SD card. The communication interface 7 may be e.g. a Bluetooth transmitter, WiFi transmitter etc. Though it is not shown in Figure 1A, the portable measuring device also comprises a power supply unit for providing power to the components of the device, and conventional electric connections between its corresponding subunits.

The filter characteristics of the first filter circuit 13a and the second filter circuit 13b are selected according to frequencies of electrical signals emitted from different internal organs or different groups of organs. For example, the first filter characteristics of the first filter circuit 13a are adapted for separation of electric signals emanating from heart muscles, i.e. for separation of ECG signals, while the second filter characteristics of the second filter circuit 13b are adapted for separation of electric signals emanating from smooth muscle tissue of organs of the visceral region, i.e. for separation of EGIG signals. As the frequency of the ECG signals is higher than that of the EGIG signals, the first filter circuit 13a is preferably a high-pass filter, e.g. with a cutoff frequency of 0.5 Hz, while the second filter circuit 13b is preferably a low-pass filter, e.g. with a cutoff frequency of 0.3 Hz. A further option is to configure at least one of, preferably both of the two filter circuits 13a, 13b as band-pass filters, e.g. the filter circuit 13a with a pass band from 0.5 Hz to 1000 Hz and the filter circuit 13b with a pass band from 0.016 Hz to 0.3 Hz. The above boundary frequencies of the filter circuits 13a, 13b are merely examples that provide a confident separation of the ECG and EGIG signals from each other, the boundary frequencies may be selected according to the electric activity of the organs to be examined in a manner known to a person skilled in the art.

The data about the electric activity of the internal organs provided by the portable measuring device according to the invention is more accurate than that of the prior art solutions and is also phase correct (or simultaneously recorded). This is the result of the fact, that the portable measuring device according to the invention separates the electric signals emitted by the organs or organ groups by analogue filtering before the digitalization - contrary to the usual course of signal processing -, substantially without phase distortion, immediately after amplification of the signals detected by the electrodes 11a, 11b.

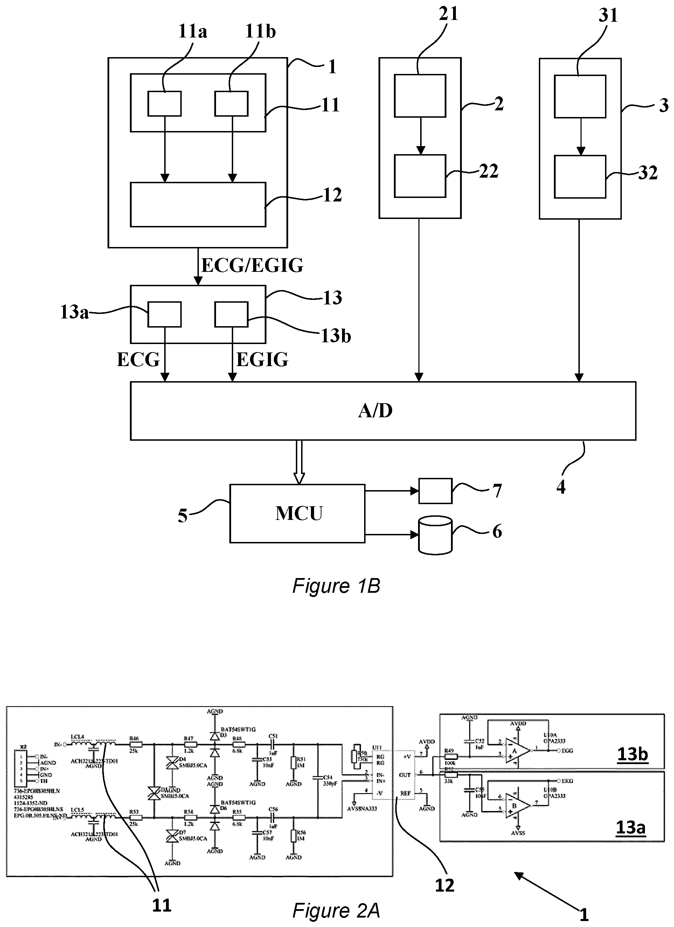

The portable measuring device according to the invention preferably comprises one or more measurement units for measuring further parameters beside ECG and EGIG signals, particularly at least one of a body temperature measurement unit 2 and a GSR measurement unit 3, as shown in Figure 1B. The body temperature measurement unit 2 comprises a temperature sensor 21 suitable for placement on the body of the patient and a signal amplifier 22 connected thereto. The GSR measurement unit 3 comprises a GSR sensor 31 for placement on the body of the patient and a signal amplifier 32 connected thereto. The outputs of the body temperature measurement unit 2 and the GSR measurement unit 3 are connected to the A/D converter unit 4. It is important for the invention that the further optional measurement units transfer the electric signals of their sensors to the A/D converter unit 4 substantially without delay so that the corresponding physiological signals also stay in correct phase with the EGIG/ECG signals detected by the electrodes 11a, 11b.

Figure 2A shows a circuit diagram of the measurement unit 1 in a preferred embodiment of the portable measuring device according to the invention. In the Figure, the electric components are represented by their conventionally used symbols known to the skilled person. The electrode pair 11 is connected to the signal amplifier 12 via suitable interposed components known to a person skilled in the art, wherein the signal amplifier 12 is preferably an instrumentation amplifier. The output of the signal amplifier 12 is connected to the first filter circuit 13a for separating the ECG signals and to the second filter circuit 13b for separating the EGIG signals.

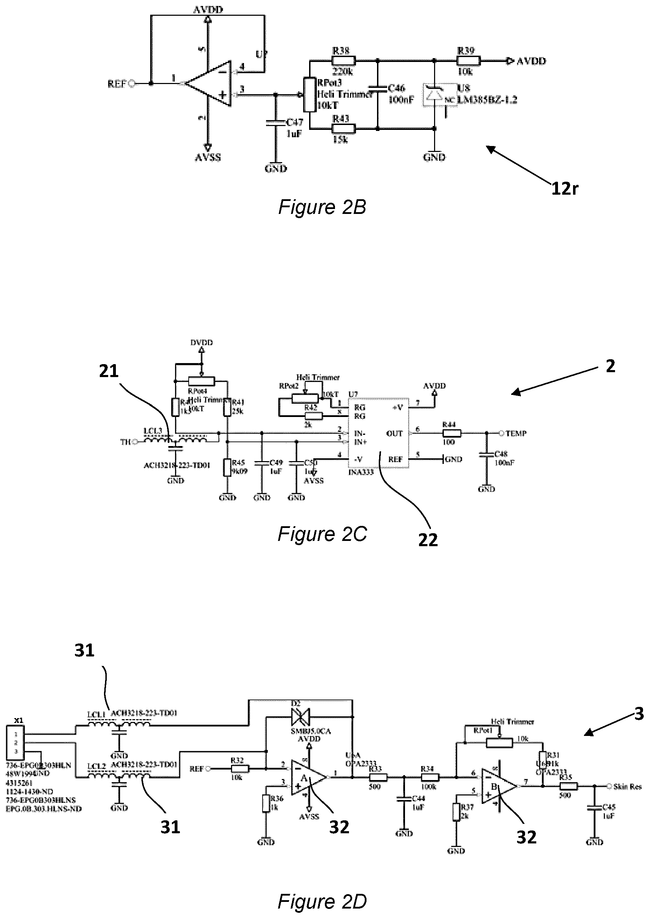

Figure 2B shows a circuit diagram of an artificial ground circuit 12r used in a preferred embodiment of the portable measuring device according to the invention. When measuring electrical signals emanating from human or animal body, e.g. myoelectric signals, a significant problem is to provide a reference potential - i.e. a ground - that makes it possible to measure the signals with low noise. This is usually accomplished by placing a third electrode on one of the limbs, but wearing such an electrode and the associated wiring increases discomfort of the patient. It is possible to use a real ground, but it would cause too much noise in the measurements. By the use of the artificial ground circuit 12r shown in Figure 2B the noise of the myoelectric measurements may be reduced without increasing the discomfort caused to a patient by wearing of the portable measuring device. The use of such an artificial ground circuit 12r is particularly preferred in an embodiment of the portable measuring device that comprises a GSR measurement unit 3, because GSR measurement includes conducting direct current into the skin for measuring its electric resistance, which reduces the accuracy of the myoelectric measurements or possibly even makes it impossible to evaluate the measurement results if the null point is shifted by so much that the analogue-digital converter(s) is/are constantly saturated even without actual myoelectric signal.

A preferred solution of this problem is using the artificial ground circuit 12r shown in Figure 2B, which provides an artificial electronic reference point for every such analogue electric component of the portable measuring device that would require connecting one of its terminals to the ground. The artificial ground circuit 12r is preferably formed as a part of the signal amplifier 12, i.e. the *REF* point shown in Figure 2B is the same as the *AGND* point connected to the *REF* output of the signal amplifier 12 in Figure 2A. As shown in Figure 2B, the artificial ground circuit 12r preferably comprises a voltage stabilizer stage, a voltage divider stage connected to the voltage stabilizer stage, and a symmetrically supplied amplifier connected to the voltage divider stage, preferably in a voltage follower connection. Naturally, the artificial ground circuit 12r may be formed as a unit separate from the signal amplifier 12, and with a circuitry different from that of the embodiment shown in Figure 2B.

Figures 2C and 2D respectively show circuit diagrams of the body temperature measurement unit 2 and the GSR measurement unit 3 preferably used in the portable measuring device according to the invention. When equipped with the body temperature measurement unit 2 and the GSR measurement unit 3, the portable measuring device according to the invention is capable of providing data about more physiological parameters, thus even further facilitating the observation of the medical conditions of a patient and identification of pathological conditions.

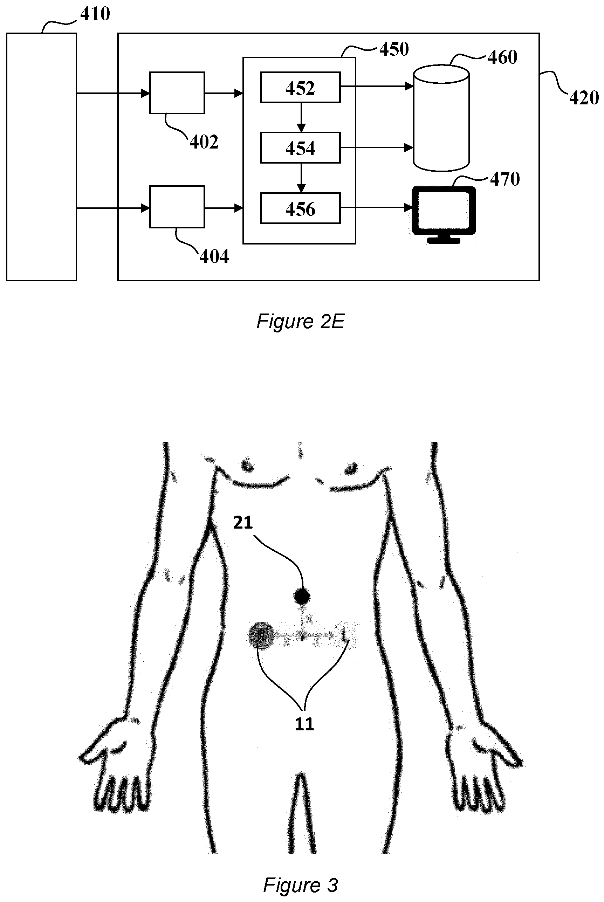

Figure 2E shows a functional block diagram of the medical diagnostic system 400 according to the invention. One of the main components of the system 400 is a measuring device 410 as described in detail above in relation with Figures 1A and 1B, and a further main component of the system 400 is a central computer 420. Data from the measuring device 410 is transferrable to the central computer 420 via a wired or wireless connection, e.g. Bluetooth or Wi-Fi, or by a portable data storage medium. Accordingly, the central computer 420 comprises a wired or wireless communication interface 402 or a data input device 404, which may be formed by e.g. an SD card reader or a USB port.

The central computer 420 may be a desktop computer (PC), laptop, tablet or even a smartphone.

The central computer 420 comprises a data processing unit 450 having a processor and comprising an evaluation module 452, a data storage module 454 and a display module 456.

The evaluation module 452 automatically determines different diagnostic data from the obtained digital data of the primary curves. It performs RSA (Running Spectral Analysis), preferably by using Fast Fourier Transform (FFT), on the EGIG curve with a predetermined window time (typically 3-15 minute window times) for obtaining a power spectrum, amplitude spectrum and several different frequency values, e.g. PS, MA, CPM for each organ of the gastrointestinal (GI) region. It also produces a trend from the Heart Rate (HR), and evaluates Heart Rate Variability (HRV) on a time/frequency basis. It also produces time based trends from the TH curves provided by the body temperature measurement unit and the GSR curves. The evaluation module 452 preferably determines time passing between local extremums on a finite section of at least one of the EGIG and ECG curves, and optionally the average and/or variance thereof.

The evaluated EGIG data may be displayed in a 2D or 3D format, while HRV data may be displayed in a spectral diagram by the display module 456 on a monitor 470. The evaluated data and their graphical representations may be exported in CSV format to a local data storage 460.

In a further step, the evaluation module 452 determines the nature and extent of possible pathological abnormalities from the preprocessed data by a built-in diagnostic algorithm. The basis of the diagnostic algorithm is formed by known statistic methods (e.g. CHAID decision tree, ANOVA test, Bonferroni test, logistic regression verified by ROC analysis etc.).

Elimination of possible random artifacts caused by the movement of the patient (or the examined animal) or the faulty fixing of electrodes may be carried out during the diagnostic evaluation. One possible way to eliminate these errors is the limitation of the voltage level of the primary curves, and another possible way is to use a statistical IQR (interquartile range) filtering in a second evaluation step. The latter procedure is based on determining the interquartile range of the total power (PS) data of the complete measurement range, and keeping only the data falling within this interquartile range.

The data produced by the evaluation module 452 during data processing are stored in the data storage unit 454. The diagnostic results produced during data processing are displayed by the display module 456 in a graphic form and/or as a table and/or as text on a monitor 470. The temporally changing wave forms of the different physiological signals provided by the measuring device 410 may also be displayed on the monitor 470.

The diagnostic data calculated by the evaluation module 452, and the Holter-type data records formatted by the data storage module 454 are placed in the local data storage 460 of the central computer 420 for persistent storage, e.g. in CHF format. The wave forms of the physiological signals received from the measuring device 410 are also stored in the local data storage 460 with real time stamps.

In what follows, the main steps of a diagnostic method not covered by the claimed invention are described. The diagnostic method is performed by the medical diagnostic system according to the invention as described above.

As a first step of the diagnostic method, the electrodes for simultaneous measuring of the EGIG and ECG signals are placed on the patient (or on the animal to be examined), and in a preferred variant of the method, also placing body temperature measuring and/or GSR measurement sensors. Figure 3 shows a preferred arrangement of the sensors on the body of the patient, with the electrode pair 11 for measuring the EGIG and ECG signals and the temperature measurement sensor 21 arranged at equal distances from the umbilicus. In order to make the examinations performed by the measuring device as non-invasive as possible, and to cause as little discomfort as possible, the electrodes and sensors used for the measurements are preferably adhered to the skin.

The next step of the method is measuring different physiological signals of the patient simultaneously by the electrode pair and optionally by additional sensors, e.g. body temperature measurement unit and GSR measurement unit, for a predetermined duration. The measured physiological signals are at least the EGIG and ECG signals of the patient, and according to the actual needs, for providing even more accurate diagnosis, the body temperature and the galvanic skin response may also be measured. For a predetermined period during the measurement, every measured physiological signal is detected simultaneously by the electrodes and optionally by further sensors.

Performing measurements for several hours or even 1 day with suitable accuracy requires prevention of movement of electrodes and of optional further sensors on the surface of the body. The physical parameters of the electric connection between the skin and the electrodes (and optionally other sensors) also needs to be kept unchanged. A preferred solution for this problem is using a medical gel. In order to find a suitable electrode-gel pair for this purpose, the long-term stability of numerous electrodes with different materials and configurations have been tested along with numerous different gels. We have arrived to the conclusion that the EGIG/ECG electrode pair is preferably formed by Ag/AgCl electrodes, and more preferably the EGIG/ECG electrode pair is formed by sintered Ag/AgCl electrodes with a gel provided on at least a portion of their surface, wherein said gel comprises polyoxyethylene (20) cetyl ether, water, glycerol, calcium carbonate, 1,2-propanediol, potassium chloride, gelwhite, sodium chloride, polyoxyethylene (20) sorbitol, methyl paraben and propyl paraben, and wherein the chloride ion concentration of the gel is preferably at least 2 mol/dm$^3$, more preferably at least 2.2 mol/dm$^3$, most preferably 2.4 mol/dm$^3$.

In the measurement phase of the method, the portable measuring device is used for either storing the digitized signals in a format suitable for transferring later, or used for immediately transmitting the digitized signals to the central computer 420. The measurement data may be transferred to the central computer 420 via wired or wireless transfer, e.g. via Bluetooth connection, or via a portable data storage device, e.g. an SD card.

A further step of the measurement phase is using the filter unit 13 for separating the ECG and EGIG signals sensed simultaneously by the electrodes 11a, 11b. The ECG and EGIG signals are transferred to the central computer 420 separately in digitized form. When using further sensors, e.g. body temperature sensor 21 and/or GSR sensor 31, the signals of these sensors are also transferred to the central computer 420 in digital form.

In the next step of the diagnostic method, the measurement data is processed for diagnostic purpose. The details of the processing have already been described with reference to Figure 2E. During data processing, a predefined evaluation algorithm is used for determining the nature and extent of pathological or undesired physiological abnormalities present in an examined organ or organ group of the patient, especially observed in the visceral activity.

In what follows, the results of different clinical tests are described that have been performed with the use of the portable measuring device and diagnostic system according to the invention and with the diagnostic method.

In the description of the examples, the parameters and other pieces of data derived from the ECG and EGIG signals are denoted using the abbreviations conventionally used in the art, however the abbreviations and their meaning is also listed in the tables below.

Table 1: data derived from ECG

| Abbreviation | Meaning |
|---|---|
| PSD | a function showing the distribution of power over frequency (the so-called power spectral density), which is obtained by spectral analysis of the ECG signal, preferably by Fourier-transformation |
| RR interval | the distance between two consecutive "R" peaks of the ECG curve, given in msec |
| VLF power | integral of the PSD in the 0,033-0,04 Hz frequency range, given in $msec^2$ |
| LF power | integral of the PSD in the 0,04-0,15 Hz frequency range, given in $msec^2$ |
| HF power | integral of the PSD in the 0,15-0,4 Hz frequency range, given in $msec^2$ |
| total power | integral of the PSD in the 0,033-0,4 Hz frequency range, given in $msec^2$ |
| VLF% | percentage of the VLF power relative to the total power |
| LF% | percentage of the LF power relative to the total power |
| HF% | percentage of the HF power relative to the total power |
| LF/HF% | percentage of the LF power relative to the total power |
| pRR50% | percentage of consecutive NN intervals with differences larger than 50 msec relative to the total number of investigated NN interval difference,<br><br>$$100 * \left[ \frac{cnt(\sigma\|NN_{i-1} - NN_i\| > 50)}{n-1} \right]$$ |
| rMSSD | square root of the average of the squared differences of consecutive NN intervals<br><br>$$\sqrt{\left( \frac{\sum \|NN_{i-1} - NN_i\|^2}{n} \right)}$$ |
| HRVi | the quotient of the number of all of the detected RR intervals and the number of occurrences of the most often occurring RR interval<br><br>$$\frac{\sum cnt(RR_i)}{cnt\lceil mc(RR_i)\rceil}$$ |
| average HRVi, | arithmetical average of the HRVi, preferably in msec |
| heart rate variation coefficient | $$\frac{standard\ deviation\ of\ heart\ rate}{average\ of\ heart\ rate}$$ |
| CVRR% | RR interval variation coefficient given as percentage<br><br>$$\frac{standard\ deviation\ of\ RR\ intervals}{average\ of\ RR\ intervals} * 100$$ |
| average CVRR | RR interval variation coefficient<br><br>$$\frac{standard\ deviation\ of\ RR\ intervals}{average\ of\ RR\ intervals}$$ |
| average pRR50 | average of the pRR50 percentages |
| HF% variation coefficient | $$\frac{standard\ deviation\ of\ HF\%}{average\ of\ HF\%}$$ |

(continued)

| Abbreviation | Meaning |
|---|---|
| average VLF power | arithmetical average of the VLF power, preferably given in msec |

The formula of the standard deviation: $\sqrt{\dfrac{\sum(x-\overline{x})^2}{(n-1)}}$

Table 2: data derived from EGIG

| Abbreviation | Meaning |
|---|---|
| PSD | a function showing the distribution of power over frequency (the so-called power spectral density), which is obtained by spectral analysis of the EGIG signal, preferably by Fourier-transformation |
| CPM | cycles per minute, a unit of frequency, equal to 60 times Hertz |
| stomach frequency | frequency of the highest peak of the PSD in the 3-5 CPM range |
| large intestine frequency | frequency of the highest peak of the PSD in the 1-3 CPM range |
| small intestine frequency | frequency of the highest peak of the PSD in the 15-25 CPM range |
| complete range (1-25 CPM) frequency | frequency of the highest peak of the PSD in the 1-25 CPM range |
| stomach magnitude | magnitude of the highest peak of the PSD in the 3-5 CPM range |
| large intestine magnitude | magnitude of the highest peak of the PSD in the 1-3 CPM range |
| small intestine magnitude | magnitude of the highest peak of the PSD in the 15-25 CPM range |
| complete range magnitude | magnitude of the highest peak of the PSD in the 1-25 CPM range |
| stomach power | integral of the PSD in the 3-5 CPM range |
| large intestine power | integral of the PSD in the 1-3 CPM range |
| small intestine power | integral of the PSD in the 15-25 CPM range |
| complete range power | integral of the PSD in the 1-25 CPM range |
| stomach magnitude/power quotient | quotient of the stomach magnitude and the stomach power |
| small intestine magnitude/power quotient | quotient of the small intestine magnitude and the small intestine power |
| large intestine magnitude/power quotient | quotient of the large intestine magnitude and the large intestine power |

(continued)

| Abbreviation | Meaning |
|---|---|
| complete range magnitude/power quotient | quotient of the complete range magnitude and the complete range power |
| stomach magnitude/power quotient variation coefficient | quotient of the standard deviation of the stomach magnitude/power quotient and the average of the stomach magnitude/power quotient |
| stomach magnitude variation coefficient | quotient of the standard deviation of the stomach magnitude and the average of the stomach magnitude |
| small intestine magnitude variation coefficient | quotient of the standard deviation of the small intestine magnitude and the average of the small intestine magnitude |
| large intestine magnitude variation coefficient | quotient of the standard deviation of the large intestine magnitude and the average of the large intestine magnitude |
| small intestine frequency variation coefficient | quotient of the standard deviation of the small intestine frequency and the average of the small intestine frequency |
| normalized small intestine total power | $$normalized\ small\ intestine\ total\ power =$$ $$= \frac{small\ intestine\ total\ power_i - small\ intestine\ total\ power_{min}}{small\ intestine\ total\ power_{max} - small\ intestine\ total\ power_{min}}$$ wherein small intestine total power$_i$ is the i-th small intestine total power value, small intestine total power$_{min}$ is the minimum of the small intestine total power values, small intestine total power$_{max}$ is the maximum of the small intestine total power values, |
| normalized GSR | the minimum-maximum normalized GSR value: $$normalized\ GSR = \frac{GSR_i - GSR_{min}}{GSR_{max} - GSR_{min}}$$ wherein $GSR_i$ is the actual GSR value, $GSR_{min}$ is the minimum of the measured GSR valued, $GSR_{max}$ is the maximum of the measured GSR values |

As it is obvious to a person skilled in the art, any of the above quantities may be replaced with its normalized or not normalized variants, the quantities may be given in any arbitrary units with adapting the corresponding formulae accordingly, and boundaries of the given ranges are approximate values. Furthermore, the same results may be obtained from the Fourier-transformed functions, their squared variant and the PSD with corresponding corrections. The Fourier-transform is preferably performed by Fast Fourier Transform (FFT), but obviously other different suitable methods may be used similarly.

**Examples**

*Example 1 - Clinical tests of anxiety/stress and the specific secondary evaluation algorithm associated with the test, used for clearly detecting the pathological effect*

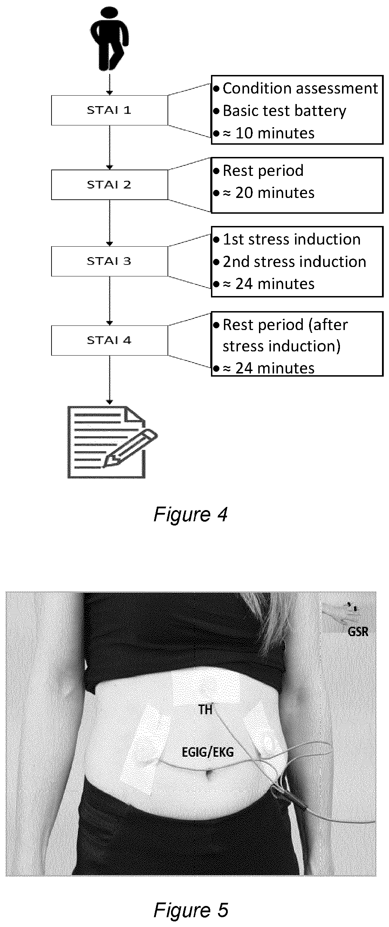

Figure 4 shows the course of the clinical test on anxiety or stress. A total of 21 healthy male and female (11 female and 10 male) voluntary participants have been subjected to the test, between ages of 20 and 26 years. The tests have been performed according to the TSST (Trier social stress test) protocol. In association with the test, State-Trait Anxiety Inventory (STAI) (developed by Spielberger et al.) have been used. During the Trier test, the state stress have been determined by STAI values on the basis of the inventory self-reported by the participant. Accordingly, the first 20 questions of the 40 questions of the self-reported inventory have been used. During the course of the TSST protocol, the STAI have been used four times.

The conductor of the test takes notes on the steps associated with the procedure into a Trier experimental journal. In order to facilitate conduction of the test and detailed objective evaluation, "Condition assessment and summary minutes" is prepared and used. The minutes include data on personal and physical conditions and the summed STAI values by each test phase. For the sake of personal data protection and to facilitate summarization of the data of the participants - with transparency kept in mind - the following codes have been implemented:

- sex: 1 male, 2 female;
- education: 1 primary, 2 secondary, 3 higher;
- smoking: 1 yes, 2 no.

[0049] Figure 5 shows the arrangement of the electrodes used for detecting anxiety or stress on a living human subject. According to the example, a pair of Ag/AgCl electrodes and a body temperature sensor (TH) are placed on the abdomen and one pair of skin resistance electrodes (GSR) is placed on the hand.

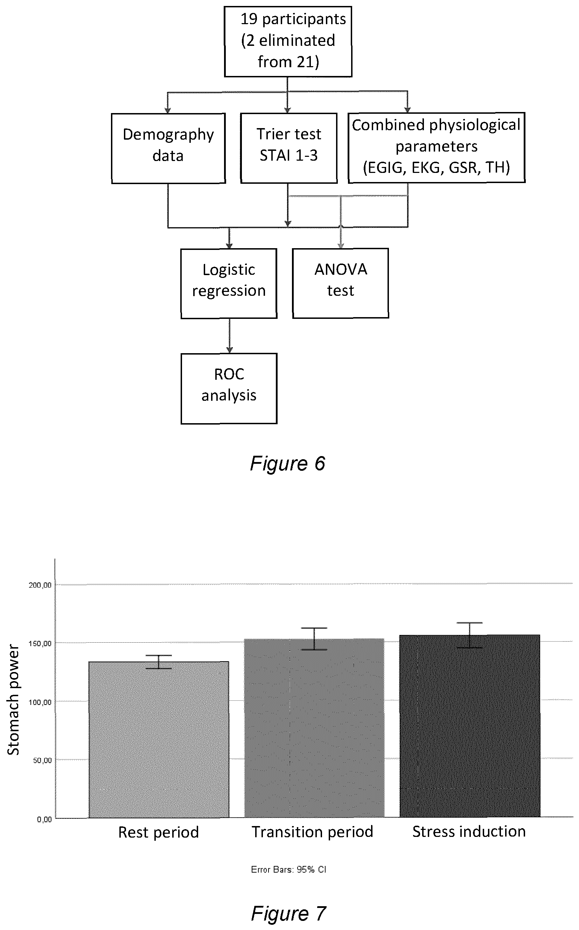

Figure 6 is a flowchart showing the course of the second stage processing used for detecting anxiety or stress. During the processing, first, the physiological data that have been evaluated is analyzed by single-factor ANOVA test individually and then Bonferroni test was used for multiple comparisons as a post-hoc test. During the processing, myoelectric waves of each organ of the GI (gastro-intestinal) region have been evaluated in individual ranges and in the complete range (see evaluation GI table below), as well as the time- and frequency based HRV derived from the ECG, and the TH and GSR data. After complementing the physiological data with demography data (from the condition assessment), the evaluation was repeated by backward conditional logistic regression. The logistic regression has been verified by ROC analysis.

Table 3: stomach total power

| (I) Section | (J) Section | Mean Difference (I-J) | Std. Error | Sig. | 95% Confidence Interval | |
| | | | | | Lower Bound | Upper Bound |
| rest period | transient period | -19.39692* | 5.52685 | .001 | -32.6809 | -6.1129 |
| | stress induction | -22.07337* | 6.05370 | .001 | -36.6237 | -7.5231 |
| transient period | rest period | 19.39692* | 5.52685 | .001 | 6.1129 | 32.6809 |
| | stress induction | -2.67646 | 7.08957 | 1.000 | -19.7165 | 14.3636 |
| stress induction | rest period | 22.07337* | 6.05370 | .001 | 7.5231 | 36.6237 |
| | transient period | 2.67646 | 7.08957 | 1.000 | -14.3636 | 19.7165 |
| * The mean difference is significant at the 0.05 level. | | | | | | |

Table 3 and the diagram shown in Figure 7 shows the total stomach power (PS) evaluation. The stomach measurement region was statistically significantly ($p<0.001$) different in all three measurement periods. The highest total power has been observed in the stress induction phase, its difference from the rest period was statistically significant ($p<0.001$) and its difference from the transition period was not statistically significant.

Table 4: small intestine total power

| (I) Section | (J) Section | Mean Difference (I-J) | Std. Error | Sig. | 95% Confidence Interval | |
| | | | | | Lower Bound | Upper Bound |
| rest period | transient period | -37.41214* | 7.22130 | .000 | -54.7688 | -20.0555 |
| | stress induction | -67.47933* | 7.90967 | .000 | -86.4905 | -48.4681 |
| transient period | rest period | 37.41214* | 7.22130 | .000 | 20.0555 | 54.7688 |
| | stress induction | -30.06718* | 9.26314 | .004 | -52.3315 | -7.8029 |
| stress induction | rest period | 67.47933* | 7.90967 | .000 | 48.4681 | 86.4905 |
| | transient period | 30.06718* | 9.26314 | .004 | 7.8029 | 52.3315 |
| * The mean difference is significant at the 0.05 level. | | | | | | |

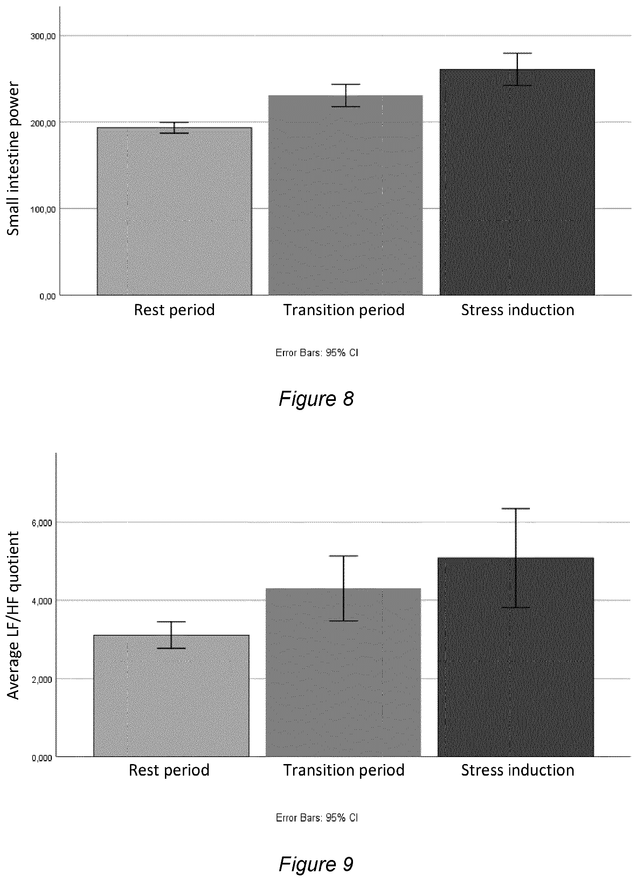

Table 4 and the diagram shown in Figure 8 shows the total small intestine power (PS) evaluation. The total power in the small intestine measurement region was statistically significantly (p<0.001) different in the three measurement periods. The highest total power has been observed in the stress induction phase, with its difference from both the rest period (p<0.001) and the transition period (p=0.004) being significant.

Table 5: frequency based HRV

| (I) Section | (J) Section | Mean Difference (I-J) | Std. Error | Sig. | 95% Confidence Interval | |
| | | | | | Lower Bound | Upper Bound |
|---|---|---|---|---|---|---|
| rest period | transient period | -1.195082* | .444116 | .022 | -2.26253 | -.12763 |
| | stress induction | -1.969601* | .486451 | .000 | -3.13881 | -.80040 |
| transient period | rest period | 1.195082* | .444116 | .022 | .12763 | 2.26253 |
| | stress induction | -.774519 | .569690 | .524 | -2.14379 | .59475 |
| stress induction | rest period | 1.969601* | .486451 | .000 | .80040 | 3.13881 |
| | transient period | .774519 | .569690 | .524 | -.59475 | 2.14379 |
| * The mean difference is significant at the 0.05 level. | | | | | | |

Table 5 and the diagram shown in Figure 9 shows the frequency based HRV evaluation (LF/HF). The LF/HF quotient was statistically significantly (p<0.001) different in the three measurement periods. The highest LF/HF quotient has been observed in the stress induction phase. The LF/HF quotient measured in the stress induction phase was statistically significantly different from the LF/HF quotient measured in the rest period (p<0.001).

Table 6: time based HRV evaluation

| (I) Section | (J) Section | Mean Difference (I-J) | Std. Error | Sig. | 95% Confidence Interval | |
| | | | | | Lower Bound | Upper Bound |
|---|---|---|---|---|---|---|
| rest period | transient period | 8.069* | 1.632 | .000 | 4.15 | 11.99 |
| | stress induction | 11.802* | 1.788 | .000 | 7.51 | 16.10 |
| transient period | rest period | -8.069* | 1.632 | .000 | -11.99 | -4.15 |
| | stress induction | 3.733 | 2.094 | .226 | -1.30 | 8.77 |
| stress induction | rest period | -11.802* | 1.788 | .000 | -16.10 | -7.51 |
| | transient period | -3.733 | 2.094 | .226 | -8.77 | 1.30 |
| * The mean difference is significant at the 0.05 level. | | | | | | |

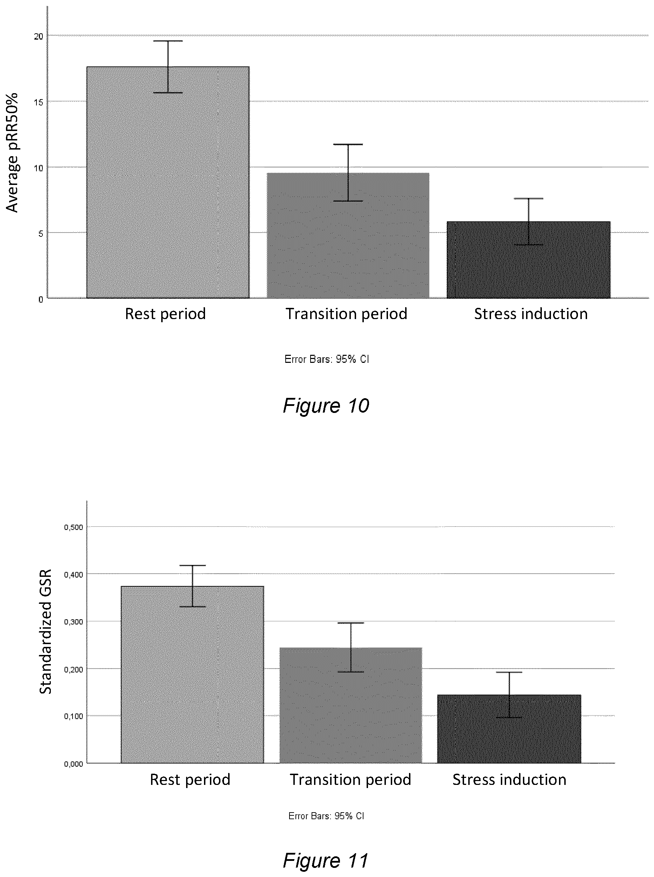

Table 6 and the diagram shown in Figure 10 shows the time based HRV evaluation (pRR50%). The pRR50% value was statistically significantly (p<0.001) different in the three measurement periods. The lowest pRR50% value has been observed in the stress induction phase. The pRR50% value measured in the stress induction phase was statistically significantly different from the pRR50% value observed in the rest period (p<0.001).

Table 7: complete range GSR (KΩ)

| (I) Section | (J) Section | Mean Difference (I-J) | Std. Error | Sig. | 95% Confidence Interval | |
| | | | | | Lower Bound | Upper Bound |
|---|---|---|---|---|---|---|
| rest period | transient period | .129404* | .037052 | .002 | .04035 | .21846 |
| | stress induction | .229629* | .040584 | .000 | .13209 | .32717 |
| transient period | rest period | -.129404* | .037052 | .002 | -.21846 | -.04035 |
| | stress induction | .100225 | .047528 | .107 | -.01401 | .21446 |

(continued)

| (I) Section | (J) Section | Mean Difference (I-J) | Std. Error | Sig. | 95% Confidence Interval | |
|---|---|---|---|---|---|---|
| | | | | | Lower Bound | Upper Bound |
| stress induction | rest period | -.229629[*] | .040584 | .000 | -.32717 | -.13209 |
| | transient period | -.100225 | .047528 | .107 | -.21446 | .01401 |
| * The mean difference is significant at the 0.05 level. | | | | | | |

Table 7 and the diagram shown in Figure 11 shows the complete range total GSR evaluation. The GSR was statistically significantly (p<0.001) different in the three measurement periods. The lowest GSR has been observed in the stress induction phase. The GSR measured in the stress induction phase was statistically significantly different from the GSR observed in the rest period (p<0.001).

Table 8: complete range TH (°C)

| (I) Section | (J) Section | Mean Difference (I-J) | Std. Error | Sig. | 95% Confidence Interval | |
|---|---|---|---|---|---|---|
| | | | | | Lower Bound | Upper Bound |
| rest period | transient period | -.278839 | .123998 | .075 | -.57687 | .01919 |
| | stress induction | -.499797[*] | .135818 | .001 | -.82624 | -.17335 |
| transient period | rest period | .278839 | .123998 | .075 | -.01919 | .57687 |
| | stress induction | -.220958 | .159059 | .497 | -.60326 | .16135 |
| stress induction | rest period | .499797[*] | .135818 | .001 | .17335 | .82624 |
| | transient period | .220958 | .159059 | .497 | -.16135 | .60326 |
| * The mean difference is significant at the 0.05 level. | | | | | | |

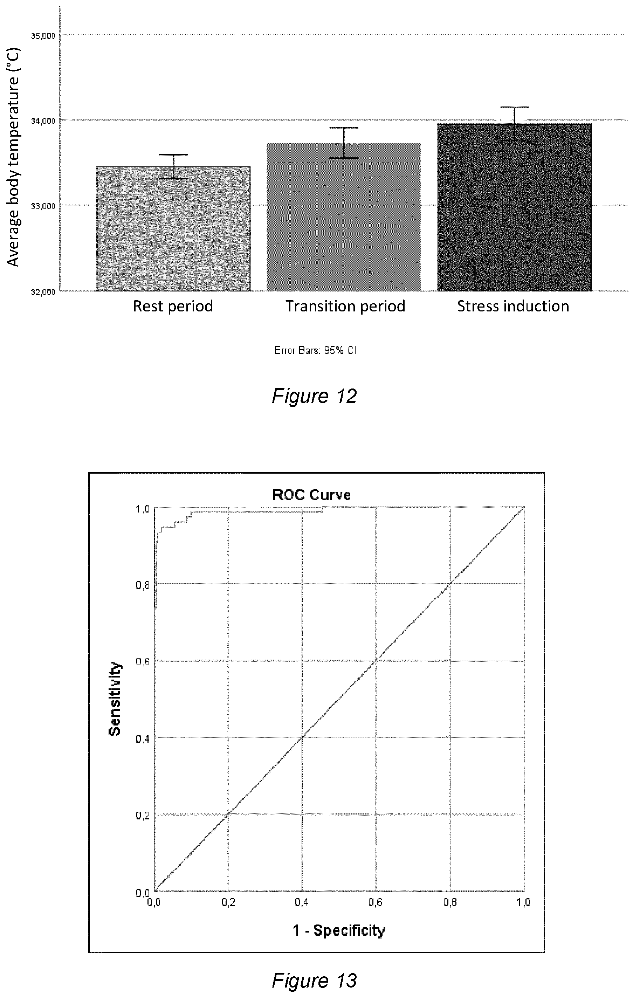

Table 8 and the diagram shown in Figure 12 shows the complete range total TH (°C) evaluation. The body temperature was statistically significantly (p<0.001) different in the three measurement periods. The temperature measured in the stress induction phase was statistically significantly different from the temperature observed in the rest period (p=0.001).

### *ROC analysis*

**Independent variables:**

- complete GI region CPM at maximum
- stomach frequency at maximum
- small intestine frequency at maximum
- large intestine frequency at maximum
- complete GI region normalized total PS
- stomach normalized total PS
- small intestine normalized total PS
- large intestine normalized total PS
- time/frequency based HRV multiple comparisons:
  average RR interval, RR interval variation coefficient (CVRR), pRR50, rMSSD, HRVi, VLF%, LF%, HF% LF/HF%
- GSR multiple comparisons
- TH multiple comparisons
- STAI values

The data has been evaluated by statistical tests (single-factor ANOVA, Bonferroni test as post-hoc test), the results have been further evaluated and verified by ROC analysis. The evaluation has been performed by backward conditional logistic regression method. The dependent variable of the regression was the section. In this case the results of the rest periods were coded with 0 value and the values of the stress induction phase were coded with 1 value.

Table 9: Logistic regression summary

| Model Summary | | | |
|---|---|---|---|
| Step | -2 Log likelihood | Cox & Snell R-Square | Nagelkerke R- Square |
| 7 | 98.241[a] | .543 | .821 |
| [a] Estimation terminated at iteration number 8 because parameter estimates changed by less than .001. | | | |
| Model Summary | | | |
| Step | -2 Log likelihood | Cox & Snell R-Square | Nagelkerke R- Square |
| 7 | 98.241[a] | .543 | .821 |
| [a] Estimation terminated at iteration number 8 because parameter estimates changed by less than .001. | | | |

Table 10: Logistic regression classification

| Classification Table[a] | | | | | |
|---|---|---|---|---|---|
| | | | Estimated | | |
| | | | Section | | |
| | Observed | | rest period | stress induction | Percentage Correct |
| Step 12 | Section | rest period | 251 | 2 | 99.2 |
| | | stress induction | 5 | 71 | 93.4 |
| | Overall Percentage | | | | 97.9 |
| [a] The cut value is .500 | | | | | |

Table 11: model variables of the logistic regression (significant variables with bold letters)

| | B | S.E. | Wald | df | Sig. | Exp(B) | 95% C.I. for EXP(B) | |
|---|---|---|---|---|---|---|---|---|
| | | | | | | | Lower | Upper |
| Large intestine normalized power | -3.239 | 1.886 | 2.949 | 1 | .086 | .039 | .001 | 1.581 |
| **small intestine normalized power** | **6.768** | **2.070** | **10.693** | **1** | **.001** | **869.898** | **15.053** | **50269.808** |
| **pRR50%** | **-.266** | **.090** | **8.693** | **1** | **.003** | **.766** | **.642** | **.915** |
| **rMSSD** | **.226** | **.045** | **24.716** | **1** | **.000** | **1.254** | **1.147** | **1.371** |
| **HRVi** | **.129** | **.049** | **7.106** | **1** | **.008** | **1.138** | **1.035** | **1.252** |
| **CVRR%** | **2.303** | **.455** | **25.628** | **1** | **.000** | **10.002** | **4.101** | **24.395** |
| **normalized GSR** | **-4.451** | **1.728** | **6.631** | **1** | **.010** | **.012** | **.000** | **.345** |
| LF/HF quotient | .155 | .092 | 2.811 | 1 | .094 | 1.167 | .974 | 1.399 |
| Constant | -113.122 | 21.737 | 27.083 | 1 | .000 | .000 | | |

Results of the tables 9, 10 and 11 may be summarized as follows. Summary of the logistic regression: the model was significant (p<0.001), the value of the Nagelkerke $R^2$ was 0.896. The variables in the regression model that significantly determine the stress induction phase are: small intestine normalized power (higher value), pRR50% (lower value), rMSSD (higher value), HRVi (higher value), CVRR% (higher value) and normalized GSR (lower value).

Table 12: AUC 0,990 (p<0.001)

| Area Under the Curve | | | | |
|---|---|---|---|---|
| | | | Asymptotic 95% Confidence Interval | |
| Area | Std. Error[a] | Asymptotic Sig.[b] | Lower Bound | Upper Bound |
| .990 | .006 | .000 | .977 | 1.000 |
| [a] Under the nonparametric assumption | | | | |
| [b] Null hypothesis: true area = 0.5 | | | | |

Table 12 and the diagram shown in Figure 13 show the results of the verification of the logistic regression by ROC analysis. The verification clearly proved that the results obtained by the secondary stage of the specific statistical evaluation of the physiological responses to the stress induction are practically equivalent to the results obtained from the self-reported STAI tests for evaluating the condition. This proved that the measuring system and method according to the invention complemented by the second stage evaluation algorithm is suitable for diagnostics in this field.

*Example 2 - Clinical tests of reflux and the specific secondary evaluation algorithm associated with the test, used for clearly detecting the pathological effect*

### INTRODUCTION

Our goal was to elaborate a non-invasive method to be used instead of the invasive pH measuring method widely used and accepted in human diagnostics in the past decades, wherein said method could clarify the basic physiological processes of the pathological abnormality and thus provide a possibility for prevention during the initial progression of the disease. This is the reason why children and young persons were selected for conducting the tests, because the pH measuring method imposes greater strain on them and the possibility of prevention is the most relevant in their cases.

### METHODOLOGY

**Test population:** 128 participants with ages between 3 and 18 years (for details see the RESULTS AND EVALUATION chapter below).

**Preliminary medical condition assessment:** general physician check-up with chemical laboratory tests.

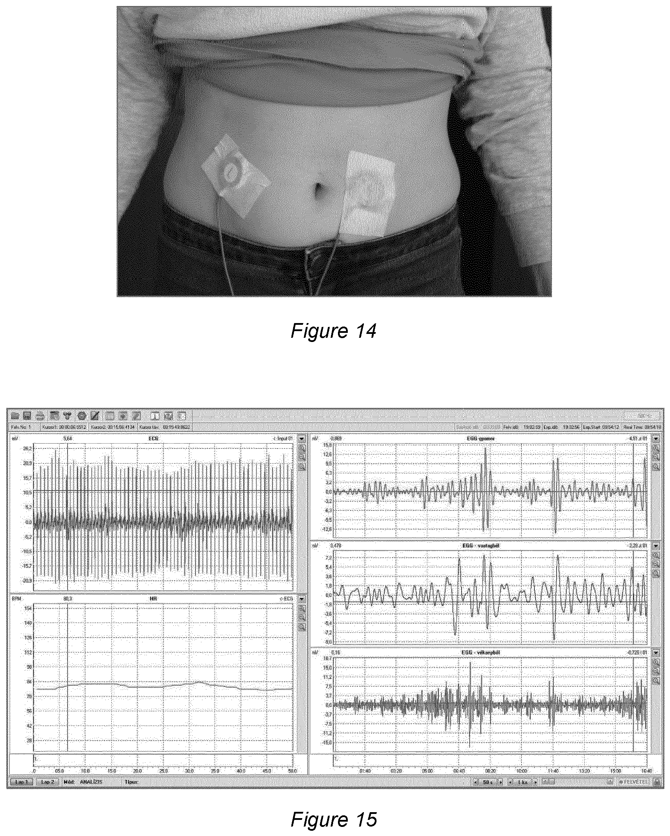

Figure 14 shows an exemplary arrangement of the electrodes on a living human subject. The general experimental arrangement for the clinical tests have been described above. In these tests only a single Ag/AgCl electrode pair has been arranged on the surface of the body.

**Measurement protocol:** EGIG/ECG Holter (with a DR4CH01 Holter/telemetry module) measurement is performed simultaneously with a 24 hour pH measurement (Menfis pHday).

**Evaluation:** Fourier-analysis (RSA) has been performed on the recorded EGIG waves and the relevant frequency (CPM) and power spectrum (PS) values have been calculated. ECG waves were used for calculating frequency based HRV values which were then statistically evaluated (for details see the RESULTS AND EVALUATION chapter below) with general physical characteristics taken into account. These statistical results and the pH statistics have been scaled to each other.

**Statistics:** CHAID decision tree method. The dependent variable of the classification was the DeMeester score determined by the pH measurement. Scores lower than 14.7 were taken as a pH negative result (i.e. not having reflux), while higher scores were taken to be pH positive (having reflux). For further details see the EVALUATION chapter below.

### RESULTS AND EVALUATION

On the basis of the condition assessment 74 participants of the total 128 participating patients had potentially reflux. The 74 participants potentially having reflux were subjected to simultaneous 24 hour EGIG/ECG and pH measurements with Holter devices. The primary EGIG/ECG waves recorded and temporarily stored by the Holter were loaded into a dedicated storage/evaluation software system according to a configuration already established in the Holter. An exemplary display of the data is shown in Figure 15.

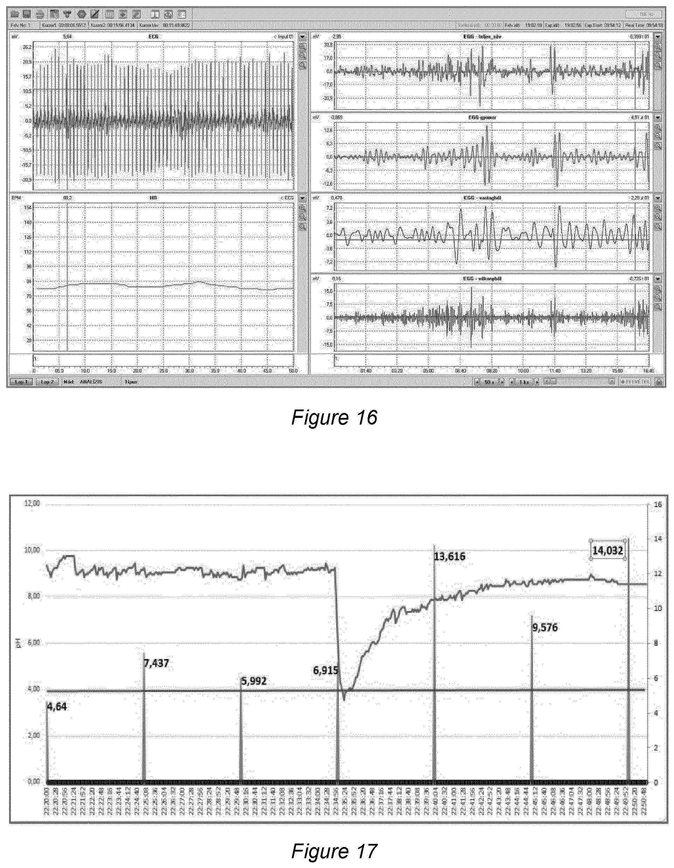

During the preparation of the evaluation of the primary waves, a need has been realized for displaying the waveform of the complete GI range (0.01 - 0.25Hz) along the primary data by default. The technical configuration of the Holter facilitates this by temporary storing the whole range (0.01 - 300Hz) of the measured waves which allowed subsequent separation of each frequency range by a software filter with high roll-off (e.g. 120 dB/decade). An exemplary display of data is shown in Figure 16.

Subsequently, the primary waves were evaluated with the method already established in the pilot studies, using the data of the frequency based HRV determined from the ECG and then the evaluated data have been recorded in a table. Figure 17 shows these results on a graph.

In the above described measurement arrangement, the experience from the pilot study have been utilized that showed that verification of a detected condition requires measurement of more than one physiological parameter pointing to the same disorder.

## *EVALUATION*

The CHAID decision tree technique has been selected for the statistical analysis of the measurement results on the grounds that a single-factor chemical parameter determined by pH measurement, the DeMeester score - that has been used as reference for detecting reflux and has been used for diagnostics for years - had to be compared with multiple-factor physiological values describing the problem. Three different models (I., II. and III.) were used for the practical implementation of the data analysis, which are described in detail together with the results in the following, after a short summary on the technique.

CHAID is a multiple-factor recursive classification technique developed by G. Kass in 1980. The technique was originally developed for variables of categorical outputs, but later the technique was improved for handling continuous parameters both as dependent variables and dependent variables. The main purpose of the explorative algorithm is to categorize the observations from the view point of the independent variable (Y) so that the variation within a category is as low as possible, while the variation between categories is as large as possible. During the use of the technique, the hierarchy of the independent variables (Chi) are also revealed on the basis of how much they explain the variance of the target variable.

As a result of these, the CHAID is a popular segmentation technique, and as such, it is a competitor for the conventional cluster analysis, which is basically only suitable for classification of observations describable by quantifiable variables. The main reason for its quick spread and popularity is that the system of connections between a selected variable and the independent variables is displayable visually in a tree structure (the decision/classification tree) that is easy to interpret. Due to the easy interpretation, the technique is particularly popular among data miners. A great advantage of the technique for making statistical models is that it does not require any limitation on the measurement scale of the variables and on their distribution, i.e. it can handle continuous and categorical dependent and independent variables ("CHAID ALAPÚ DÖNTÉSI FÁK JELLEMZŐI", Gábor HÁMORI, 2001, Statisztikai Szemle, vol. 79, issue 8).

## *Basics*

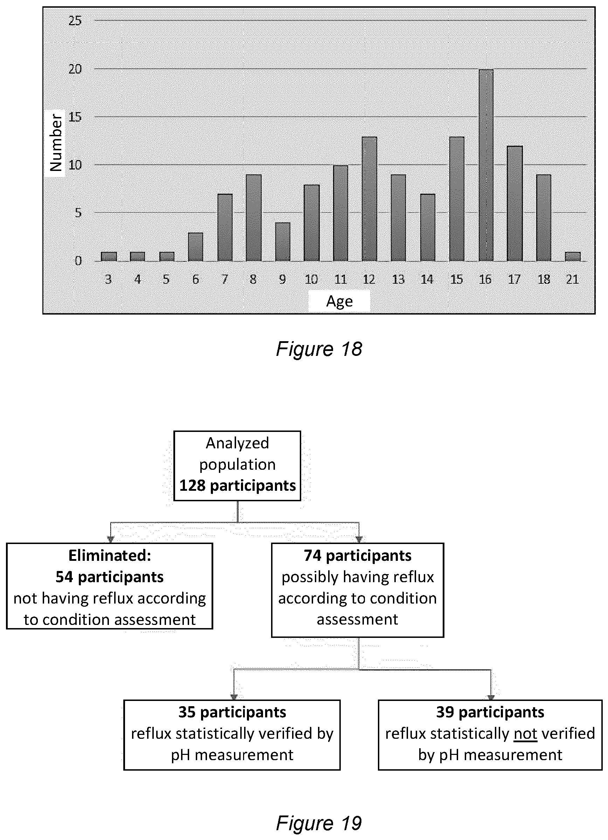

The graph shown in Figure 18 illustrates the age distribution of the 128 children and teenage participants.

Following the anamnesis and condition assessment of the 128 participants, they have been subjected to measurements by the EGIG/ECG measuring system according to the invention (from now on: EGIG/ECG). On the basis of the anamnesis and condition assessment it has been found that 74 participants are potentially having reflux, while 54 participants have been ruled out (deemed not having reflux).

The 74 participants have been subjected to simultaneous pH and EGIG/ECG measurement by Holter devices lasting 24 hours. Statistical evaluation of the pH measurement results showed 35 participants having reflux and 39 participants not having reflux. The participants having reflux had a pH statistical value, the DeMeester score > 14.7 (see the flow chart).

Figure 19 is a flow chart showing the selection and diagnosis of the persons participating in the test.

## *Initial parameters of the Models I., II. and III.*

The models I. and II. are default models for determining parameter- and algorithm bases to be used for comparative evaluation {DeMeester score (pH) vs. physiological (EGIG/EKG, etc.)} to be used in model III. The basis of both models is the examination of the 128 participants - with different selections - but in each case using the physiological tree structure compared with the DeMeester score results of the pH measurements (see flowchart). The DeMeester score > 14.7 constant value has been used for evaluation.

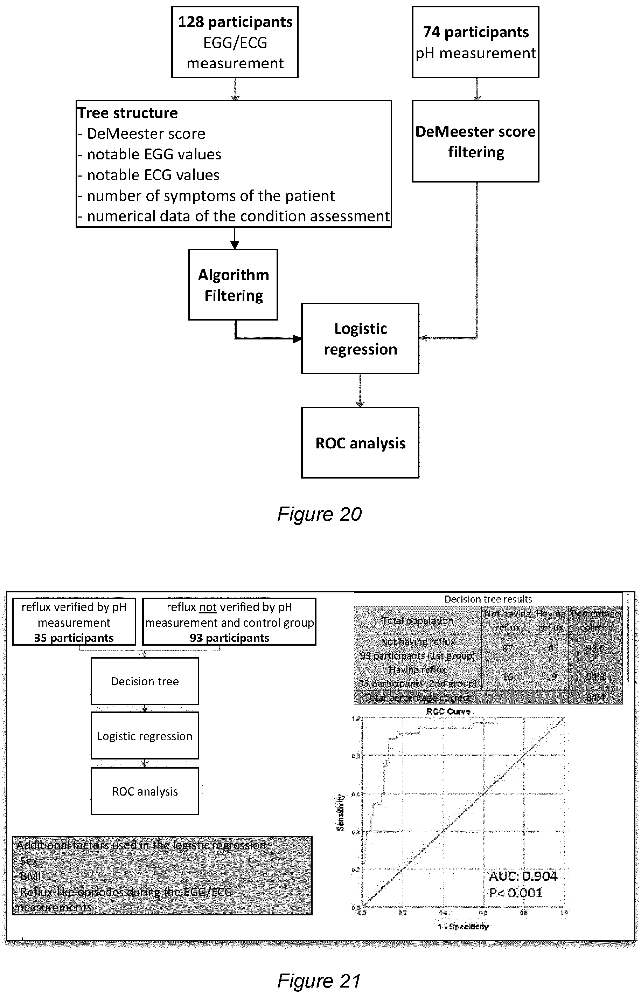

Figure 20 is a flow chart illustrating an exemplary variant of the diagnostic method according to the invention.

Algorithm: Filtering: FFT was used with 5 minute time windows for the EGIG/HRV evaluation. 24 hour recordings of the EGIG waves included artefacts that distorted the results due to their excessively large values. For the elimination of this effect, IQR statistical filtering was used such that interquartile range of the complete range total power (PS) data have been determined for each participant and only the data falling within this interquartile range have been kept.

*Model I.*

In this model two groups have been formed.

1st group: participants found not to have reflux on the basis of the condition assessment (from now on: control), and participants found not to have reflux on the basis of the DeMeester score of their pH measurement (from now on: pH-).

2nd group: participants found to have reflux on the basis of the DeMeester score of their pH measurement (from now on: pH+).

Spectral analysis has been performed on EGIG/ECG waves of both groups using FFT with 5 minute time windows. The data have been further evaluated using the above described CHAID decision tree technique. The second group with pH+ results on the basis of their DeMeester score has been taken as variable. The parameters (divided by groups) used for the evaluation are show below:

**1st GROUP (pH- and control group)**

- stomach magnitude
- large intestine magnitude
- small intestine magnitude
- complete range (1-25 CPM) power
- stomach power
- stomach magnitude/power quotient
- small intestine magnitude/power quotient
- large intestine magnitude/power quotient
- complete range (1-25 CPM) maximum CPM power%
- pRR50
- HF%
- HF power

**2nd GROUP (pH+)**

- large intestine frequency
- small intestine frequency
- RR interval
- VLF%
- VLF power
- LF%

Figure 21 shows the course of data evaluation. For participants, whose pH value had not been measured, the score value was taken to be zero. The values of the parameters of the decision tree have been evaluated by logistic regression. Further included factors are shown in the green field. Model I. recognized control participants and participants not verified by pH with a 93.5% accuracy, and recognized participants verified by pH measurement with 54.3% accuracy. The AUC value of the ROC analysis was 0.904 (p<0.001).

*Model II.*

The control group has been excluded from the two groups formed for the model and physiological parameters of the participants possibly having reflux have been examined. Accordingly, one of the groups were participants with pH- measurements and the other group was formed by those with a pH+ measurement (see below).

Spectral analysis by FFT with 5 minute time windows have been carried out on EGIG/ECG waves of both groups by a specialized software. The data groups have been further evaluated using the above described CHAID decision tree technique, wherein the dependent variable was the DeMeester score verified by the pH measurement.

**1st GROUP (pH-)**

- complete range (1-25 CPM) power
- stomach magnitude/power quotient
- small intestine magnitude/power quotient
- large intestine magnitude/power quotient
- LF%
- HF%
- HF power

**2nd GROUP (pH+)**

- stomach magnitude
- small intestine frequency
- complete range (1-25 CPM) CPM
- stomach power
- large intestine power
- small intestine power
- RR interval
- VLF%
- VLF power

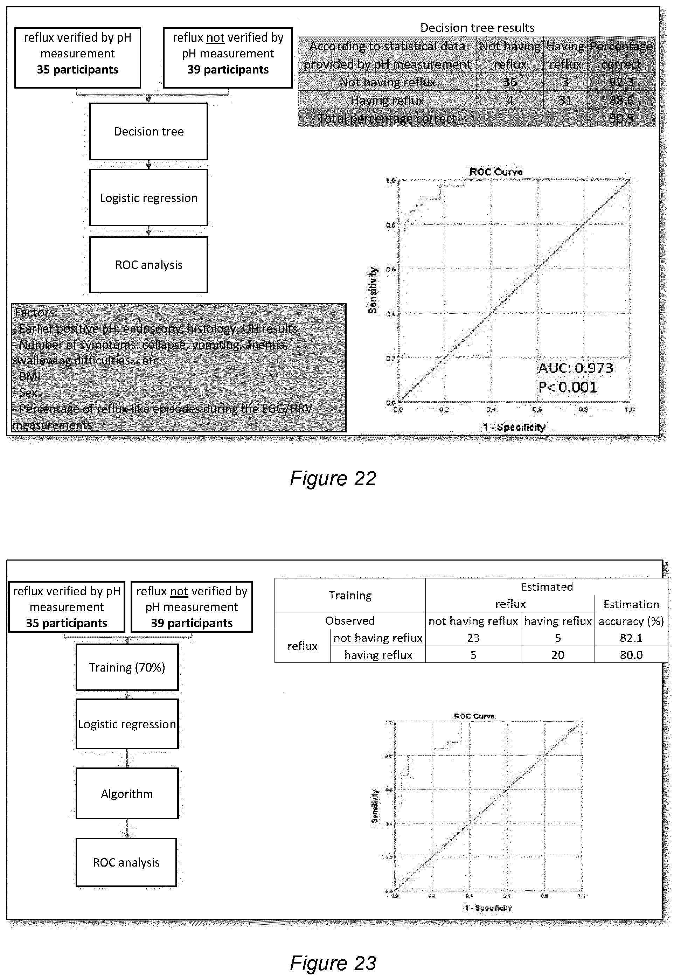

Figure 22 shows the course of the data evaluation. The parameters of the decision tree have been evaluated by logistic regression. Further included factors are shown in the green field. The model recognized participants not verified by pH with a 92.3% accuracy, and recognized participants verified by pH measurement with 88.6% accuracy. The AUC value of the ROC analysis was 0.973 (p<0.001).

*Model III.*

In this model the same 74 participants with possible reflux were evaluated as in the II. model. This model is the result of filtering the two previous models with the purpose of establishing the regression algorithm that produces the final evaluation formula. Unlike in the previous models, not separate time windows were evaluated, but instead averages across the time windows for each participant. As further derived values, variation coefficients have also been determined. The model has been formed by logistic regression. Before forming the model, "Training" and "Test" groups were created by using a random number generator. 70% of the participants (N=53) have been allocated to the Training group, while the remaining 30% (N=21) have been allocated to the Test group.

Table 13: Randomization

|  |  |  |  | N | % |
|---|---|---|---|---|---|
| Randomization | Test | having reflux? | pH negative | 11 | 52.4% |
|  |  |  | pH positive | 10 | 47.6% |
|  |  |  | Total | 21 | 100.0% |
|  | Training | having reflux? | pH negative | 28 | 52.8% |
|  |  |  | pH positive | 25 | 47.2% |
|  |  |  | Total | 53 | 100.0% |

*Training group*

The evaluation has been carried out on the randomly selected N=53 participants. The dependent variable of the regression was the DeMeester score verified by pH measurement. The list of the independent variables are as follow:

**TRAINING GROUP (pH-)**

- complete range (1-25 CPM) cycles/minute
- heart frequency average variation coefficient
- average HRVi
- average CVRR

**TRAINING GROUP (pH+)**

- stomach cycles/minute (CPM)
- stomach magnitude variation coefficient
- stomach magnitude/power quotient variation coefficient
- small intestine cycles/minute variation coefficient
- small intestine magnitude variation coefficient
- large intestine magnitude variation coefficient
- average pRR50
- average VLF power
- HF% variation coefficient

Developing the algorithm has been performed by logistic regression by using the parameters shown in Figure 22. The algorithm recognized participants not verified by pH measurement with a 82.1% accuracy and participants verified by pH measurement with 80% accuracy, the overall accuracy was 81.1%. The AUC value of the ROC analysis was 0.923 (p<0.001). The course of the evaluation is shown in Figure 23.

*Test group*

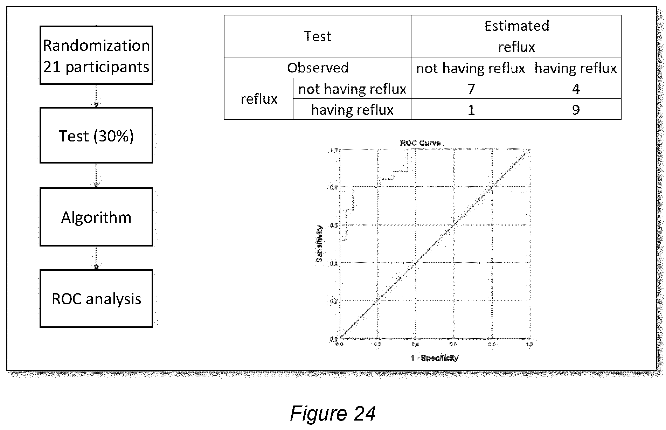

The aim of the study performed on this group is to prove the accuracy of the algorithm (used on the training group) by a pilot blind study. The result of study (for 30%, i.e. 21 participants) is that the estimation in the case of verified pH negative participants was correct for 7 participants and incorrect for 4 participants. In the case of pH positive patients, the estimation was correct for 9 participants and incorrect for 1 participant. The result of the ROC analysis was 0.845 (p=0.007). The AUC value of the ROC analysis for the joint database was 0.903 (p<0.001). The course of the evaluation is shown in Figure 24.

<u>*CONCLUSION*</u>

Comparative measurements performed for the 128 participating children and teen-age persons and the evaluation of the measurement results (pH DeMeester score > 14.7; EGIG/ECG + medical condition, from now on: EGIG/ECG+EA) verified the results and experiences of a pilot study conducted two years ago. The studies proved, that reflux may be diagnosed with 82% accuracy by using the evaluation of the data of the physiological and medical condition assessment for each individual (in comparison with reference data obtained by pH measurements in the same group). More precisely, out of 72 participants possibly having reflux, 35 participants have been diagnosed with reflux according to the pH DeMeester score > 14.7, and 29 participants have been diagnosed with reflux according to the EGIG/ECG+EA statistics (for details see the above description of clinical tests).

In order to verify the positive results, a blind test is proposed with an identical number of participants as the studies above, who had not been previously diagnosed. The organization of such a study is in progress, the necessary permission from the national Institute of Pharmacy and Nutrition has been obtained. If the blind test studies are successful, the diagnostics may be made available widely both for prevention (recognition of the disease in an early stage, pharmacological research etc.) and rehabilitation.

**Claims**

1. System (400) for detecting physiological changes in the visceral activity, for detecting physiological conditions associated with said change and optionally for diagnosing an undesired or pathological condition associated with said change, wherein said system comprises:

   - a portable measuring device (410) for measuring temporal change of physiological signals emanating from human or animal muscle tissue,

wherein the measuring device (410) comprises:

- a measurement unit (1), comprising:

- at least two electrodes (11a, 11b) for detecting physiological signals emanating from muscle tissue;
- a broadband signal amplifier (12) connected to the electrodes (11a, 11b);
- a filter unit (13) connected to an output of the broadband signal amplifier (12);
- an A/D converter unit (4) connected to an output of the filter unit (13), and
- a microcontroller unit (5) connected to an output of the A/D converter unit (4),

wherein each of the at least two electrodes (11a, 11b) are configured for simultaneously detecting electrogastrointestinography, EGIG, and electrocardiogram, ECG, signals;
wherein the filter unit (13) comprises a first filter circuit (13a) having first filter characteristics for separating the ECG signal from the input signal and a second filter circuit (13b) having second filter characteristics different from the first filter characteristics for separating the EGIG signal from the input signal, wherein the outputs of the first filter circuit (13a) and the second filter circuit (13b) are separately connected to the A/D converter unit (4); and
wherein the A/D converter unit (4) converts the analogue EGIG and ECG signals to digital signals concurrently;

- a central computer (420) in data communication with the measuring device (410), wherein said central computer (420) comprises:

- a wired or wireless communication interface (402);
- a data input device (404);
- a data processing unit (450) having a processor;
- a data storage (460); and preferably
- a monitor (470);

wherein the data processing unit (450) comprises:

- an evaluation module (452) for detecting changes in the visceral activity of a patient by a predetermined algorithm on the basis of at least the digitalized ECG and EGIG signals as input data and for detecting physiological conditions associated with said changes;
- a data storage module (454) for storing output data concerning a physiological condition that is produced by the evaluation module (452) into the data storage (460); and
- a display module (456) for displaying measurement data from the measuring device (410) and for displaying on the monitor (470) said output data produced by the evaluation module (452) concerning a physiological condition
- wherein said predetermined algorithm is selected from the group of ANOVA test, Bonferroni test and logistic regression for the ECG signal processing, and the CHAID *(Chi-square automatic interaction detection)* decision tree technique for the EGIG signal processing.

**2.** System according to claim 1, **characterized in that** the processor is configured to determine the interquartile range of the total power (PS) data on the total measurement range, and to only use measured values corresponding to this range for producing derived data.

**3.** System according to claim 1 or 2, **characterized in that** the evaluation module (452) is configured to produce at least one of, preferably all of the following parameters from first digital signals formed by ECG signals:

the distance between two consecutive "R" peaks of the ECG curve, RR interval, heart rate average variation coefficient,
the quotient of the number of all of the detected RR intervals and the number of occurrences of the most often occurring RR interval, average HRVi,
RR interval variation coefficient, average CVRR,
average of the pRR50 percentages,
integral of the power spectral density function, PSD, in the 0,15-0,4 Hz frequency range, HF power, percentage of the HF power relative to the total power, HF%, ratio of the standard deviation of HF% to the average of HF%, HF% variation coefficient,
integral of the PSD in the 0,04-0,15 Hz frequency range, LF power,
percentage of the LF power relative to the total power, LF%,
percentage of the VLF power relative to the total power, VLF%,
integral of the PSD in the 0,033-0,04 Hz frequency range, VLF power, arithmetical average of the VLF power,
average VLF power,
and optionally
to produce at least one of, preferably all of the following parameters from second digital signals formed by EGIG
signals:

> stomach magnitude,
> large intestine magnitude,
> small intestine magnitude,
> stomach power,
> small intestine power,
> large intestine power,
> stomach magnitude/power quotient,
> small intestine magnitude/power quotient,
> large intestine magnitude/power quotient,
> integral of the PSD in the 1-25 cycles per minute, CPM, range, complete range power,
> complete range (1-25 CPM) magnitude/power quotient,
> stomach frequency,
> small intestine frequency,
> large intestine frequency,
> complete range (1-25 CPM) frequency,
> stomach magnitude variation coefficient,
> stomach magnitude/power quotient variation coefficient,
> small intestine frequency variation coefficient,
> small intestine magnitude variation coefficient,
> large intestine magnitude variation coefficient.

4. System according to any one of claims 1 to 3, **characterized in that** the processor is configured to determine an indicator associated with a physiological condition of the patient, particularly an indicator indicative of psychological stress or anxiety or an indicator indicative of gastro-esophageal reflux disease, on the basis of the derived data.

5. System according to claim 4, **characterized in that** the indicator indicative of psychological stress or anxiety is determined on the basis of at least one of the following derived data:

> normalized small intestine total power,
> pRR50%,
> square root of the average of the squared differences of consecutive NN intervals, rMSSD,
> the quotient of the number of all of the detected RR intervals and the number of occurrences of the most often occurring RR interval, HRVi, RR interval variation coefficient, CVRR%, normalized galvanic skin response, normalized GSR).

**Patentansprüche**

1. System (400) zum Erkennen physiologischer Veränderungen der viszeralen Aktivität, zum Erkennen physiologischer Zustände, die mit der Veränderung assoziiert sind, und gegebenenfalls zum Diagnostizieren eines unerwünschten oder pathologischen Zustands, der mit der Veränderung assoziiert ist, wobei das System umfasst:

- eine tragbare Messvorrichtung (410) zum Messen zeitlicher Veränderungen physiologischer Signale, die von menschlichem oder tierischem Muskelgewebe ausgehen, wobei die Messvorrichtung (410) umfasst:

- eine Messeinheit (1), umfassend:

- mindestens zwei Elektroden (11a, 11b) zum Erkennen physiologischer Signale, die von Muskelgewebe ausgehen;
- einen Breitbandsignalverstärker (12), der mit den Elektroden (11a, 11b) verbunden ist;
- eine Filtereinheit (13), die mit einem Ausgang des Breitbandsignalverstärkers (12) verbunden ist;
- eine A/D-Wandlereinheit (4), die mit einem Ausgang der Filtereinheit (13) verbunden ist, und
- eine Mikrosteuereinheit (5), die mit einem Ausgang der A/D-Wandlereinheit (4) verbunden ist,

wobei jede der mindestens zwei Elektroden (11a, 11b) zum gleichzeitigen Erkennen von Elektrogastrointestinographie-, EGIG-, und Elektrokardiogramm-, EKG-, Signalen konfiguriert ist;
wobei die Filtereinheit (13) eine erste Filterschaltung (13a), die erste Filtereigenschaften zum Trennen des EKG-Signals vom Eingangssignal aufweist, und eine zweite Filterschaltung (13b) umfasst, die sich von den ersten Filtereigenschaften unterscheidende zweite Filtereigenschaften zum Trennen des EGIG-Signals vom Eingangssignal aufweist,
wobei die Ausgänge der ersten Filterschaltung (13a) und der zweiten Filterschaltung (13b) getrennt mit der A/D-Wandlereinheit (4) verbunden sind; und
wobei die A/D-Wandlereinheit (4) die analogen EGIG- und EKG-Signale gleichzeitig in digitale Signale umwandelt;

- einen Zentralcomputer (420) in Datenkommunikation mit der Messvorrichtung (410), wobei der Zentralcomputer (420) umfasst:

- eine drahtgebundene oder drahtlose Kommunikationsschnittstelle (402);
- eine Dateneingabevorrichtung (404);
- eine Datenverarbeitungseinheit (450), die einen Prozessor aufweist;
- einen Datenspeicher (460); und vorzugsweise
- einen Monitor (470);

wobei die Datenverarbeitungseinheit (450) umfasst:

- ein Auswertungsmodul (452) zum Erkennen von Veränderungen der viszeralen Aktivität eines Patienten durch einen vorbestimmten Algorithmus auf Basis mindestens der digitalisierten EKG- und EGIG-Signale als Eingangsdaten und zum Erkennen physiologischer Zustände, die mit den Veränderungen assoziiert sind;
- ein Datenspeichermodul (454) zum Speichern von Ausgabedaten bezüglich eines physiologischen Zustands, die vom Auswertungsmodul (452) erzeugt werden, im Datenspeicher (460); und
- ein Anzeigemodul (456) zum Anzeigen von Messdaten von der Messvorrichtung (410) und zum Anzeigen der vom Auswertungsmodul (452) erzeugten Ausgabedaten bezüglich eines physiologischen Zustands auf dem Monitor (470),

- wobei der vorbestimmte Algorithmus ausgewählt ist aus der Gruppe von ANOVA-Test, Bonferroni-Test und logistischer Regression für die EKG-Signalverarbeitung, und der CHAID- (Chi-square Automatic Interaction Detection) Entscheidungsbaumtechnik für die EGIG-Signalverarbeitung.

2. System nach Anspruch 1, **dadurch gekennzeichnet, dass** der Prozessor so konfiguriert ist, dass er den Interquartilbereich der Gesamtleistungsdaten (PS) im gesamten Messbereich bestimmt und nur Messwerte, die diesem Bereich entsprechen, zum Erzeugen abgeleiteter Daten verwendet.

3. System nach Anspruch 1 oder 2, **dadurch gekennzeichnet, dass** das Auswertungsmodul (452) so konfiguriert ist, dass es aus ersten digitalen Signalen, die von EKG-Signalen gebildet werden, mindestens einen, vorzugsweise alle der folgenden Parameter erzeugt:

den Abstand zwischen zwei aufeinanderfolgenden "R"-Spitzen der EKG-Kurve, RR-Intervall, den durchschnittlichen Variationskoeffizienten der Herzfrequenz,
den Quotienten der Anzahl aller erkannten RR-Intervalle und der Anzahl von Vorkommnissen des am häufigsten vorkommenden RR-Intervalls, durchschnittlicher HRVi, Variationskoeffizient des RR-Intervalls, durchschnittliche CVRR, Durchschnitt der pRR50-Prozentsätze,
Integral der spektralen Leistungsdichtefunktion, PSD, im Frequenzbereich 0,15-0,4 Hz, HF-Leistung, Prozentsatz der HF-Leistung relativ zur Gesamtleistung, HF%, Verhältnis der Standardabweichung von HF% zum Durchschnitt von HF%, HF%-Variationskoeffizient, Integral der PSD im Frequenzbereich 0,04-0,15 Hz, LF-Leistung, Prozentsatz der LF-Leistung relativ zur Gesamtleistung, LF%, Prozentsatz der VLF-Leistung relativ zur Gesamtleistung, VLF%, Integral der PSD im Frequenzbereich 0,033-0,04 Hz, VLF-Leistung, arithmetischer Durchschnitt der VLF-Leistung, durchschnittliche VLF-Leistung, und gegebenenfalls

aus zweiten digitalen Signalen, die von EGIG-Signalen gebildet werden, mindestens einen, vorzugsweise alle der folgenden Parameter erzeugt:

Magenmagnitude,
Dickdarmmagnitude,
Dünndarmmagnitude,
Magenleistung,
Dünndarmleistung,
Dickdarmleistung,
Quotient Magenmagnitude/-leistung,
Quotient Dünndarmmagnitude/-leistung,
Quotient Dickdarmmagnitude/-leistung,
Integral der PSD im Bereich von 1-25 Zyklen pro Minute, CPM, Leistung im gesamten Bereich,
Quotient Magnitude/Leistung im gesamten Bereich (1-25 CPM),
Magenfrequenz,
Dünndarmfrequenz,
Dickdarmfrequenz,
Frequenz im gesamten Bereich (1-25 CPM),
Variationskoeffizient der Magenmagnitude,
Variationskoeffizient des Quotienten Magenmagnitude/-leistung,
Variationskoeffizient der Dünndarmfrequenz,
Variationskoeffizient der Dünndarmmagnitude,
Variationskoeffizient der Dickdarmmagnitude.

4. System nach einem der Ansprüche 1 bis 3, **dadurch gekennzeichnet, dass** der Prozessor so konfiguriert ist, dass er auf Basis der abgeleiteten Daten einen Indikator bestimmt, der mit einem physiologischen Zustand des Patienten assoziiert ist, insbesondere einen Indikator, der auf psychischen Stress oder Angst hinweist, oder einen Indikator, der auf gastroösophageale Refluxkrankheit hinweist.

5. System nach Anspruch 4, **dadurch gekennzeichnet, dass** der Indikator, der auf psychischen Stress oder Angst hinweist, auf Basis mindestens einer der folgenden abgeleiteten Daten bestimmt wird:

normalisierte Gesamtleistung des Dünndarms,
pRR50%,
Quadratwurzel des Durchschnitts der quadrierten Differenzen aufeinanderfolgender NN-Intervalle, rMSSD, der Quotient der Anzahl aller erkannten RR-Intervalle und der Anzahl von Vorkommnissen des am häufigsten vorkommenden RR-Intervalls, HRVi, Variationskoeffizient des RR-Intervalls, CVRR%, normalisierte galvanische Hautreaktion, normalisierte GSR.

## Revendications

1. Système (400) permettant de détecter des changements physiologiques de l'activité viscérale, de détecter des conditions physiologiques associées audit changement et éventuellement de diagnostiquer une condition indésirable ou pathologique associée audit changement, dans lequel ledit système comprend :

- un dispositif de mesure portable (410) pour mesurer un changement temporel de signaux physiologiques émanant de tissus musculaires humains ou animaux, dans lequel le dispositif de mesure (410) comprend :

- une unité de mesure (1), comprenant :

- au moins deux électrodes (11a, 11b) pour détecter des signaux physiologiques émanant de tissus musculaires ;
- un amplificateur de signal à large bande (12) connecté aux électrodes (11a, 11b) ;
- une unité de filtrage (13) connectée à une sortie de l'amplificateur de signal à large bande (12) ;
- une unité de conversion analogique-numérique, A/N, (4) connectée à une sortie de l'unité de filtrage (13), et
- une unité de microcontrôleur (5) connectée à une sortie de l'unité de conversion A/N (4),

dans lequel chacune des au moins deux électrodes (11a, 11b) est configurée pour détecter simultanément des signaux d'électrogastro-intestinographie, EGIG, et d'électrocardiogramme, ECG ;
dans lequel l'unité de filtrage (13) comprend un premier circuit de filtrage (13a) présentant des premières caractéristiques de filtrage pour séparer le signal ECG du signal d'entrée et un second circuit de filtrage (13b) présentant des secondes caractéristiques de filtrage différentes des premières caractéristiques de filtrage pour séparer le signal EGIG du signal d'entrée,
dans lequel les sorties du premier circuit de filtrage (13a) et du second circuit de filtrage (13b) sont connectées séparément à l'unité de conversion A/N (4) ; et
dans lequel l'unité de conversion A/N (4) convertit simultanément les signaux EGIG et ECG analogiques en signaux numériques ;

- un ordinateur central (420) en communication de données avec le dispositif de mesure (410), dans lequel ledit ordinateur central (420) comprend :

- une interface de communication filaire ou sans fil (402) ;
- un dispositif d'entrée de données (404) ;
- une unité de traitement de données (450) présentant un processeur ;
- un stockage de données (460) ; et de préférence
- un moniteur (470) ;

dans lequel l'unité de traitement de données (450) comprend :

- un module d'évaluation (452) pour détecter des changements de l'activité viscérale d'un patient par un algorithme prédéterminé sur la base d'au moins les signaux ECG et EGIG numérisés comme données d'entrée et pour détecter des conditions physiologiques associées auxdits changements ;
- un module de stockage de données (454) pour stocker des données de sortie concernant une condition physiologique qui est produite par le module d'évaluation (452) dans le stockage de données (460) ; et
- un module d'affichage (456) pour afficher des données de mesure provenant du dispositif de mesure (410) et pour afficher, sur le moniteur (470), lesdites données de sortie produites par le module d'évaluation (452) concernant une condition physiologique,

- dans lequel ledit algorithme prédéterminé est choisi dans le groupe constitué du test ANOVA, du test de Bonferroni et d'une régression logistique pour le traitement de signal ECG, et de la technique d'arbre de décision CHAID (détection automatique d'interaction du Chi-carré) pour le traitement de signal EGIG.

2. Système selon la revendication 1, **caractérisé en ce que** le processeur est configuré pour déterminer la plage interquartile des données de puissance totale (PS) sur la plage de mesure totale, et pour n'utiliser que les valeurs mesurées correspondant à cette plage pour produire des données dérivées.

3. Système selon la revendication 1 ou 2, **caractérisé en ce que** le module d'évaluation (452) est configuré pour produire au moins un, de préférence l'ensemble des paramètres suivants à partir de premiers signaux numériques formés par des signaux ECG :

la distance entre deux pics « R » consécutifs de la courbe ECG, l'intervalle RR, le coefficient de variation moyen de la fréquence cardiaque,
le quotient du nombre de l'ensemble des intervalles RR détectés et du nombre d'occurrences de la moyenne HRVi de l'intervalle RR le plus fréquent, la moyenne CVRR du coefficient de variation de l'intervalle RR, la moyenne des pourcentages pRR50,
l'intégrale de la fonction de densité spectrale de puissance, PSD, dans la plage de fréquences 0,15-0,4 Hz, la puissance HF, le pourcentage de la puissance HF par rapport à la puissance totale, %HF, le rapport de l'écart type du %HF à la moyenne du %HF, le coefficient de variation du %HF, l'intégrale de la PSD dans la plage de fréquences 0,04-0,15 Hz, la puissance LF, le pourcentage de la puissance LF par rapport à la puissance totale, LF%, le pourcentage de la puissance VLF par rapport à la puissance totale, %VLF, l'intégrale de la PSD dans la plage de fréquences 0,033-0,04 Hz, la puissance VLF, la moyenne arithmétique de la puissance VLF, la puissance VLF moyenne et éventuellement

pour produire au moins un, de préférence l'ensemble des paramètres suivants à partir de seconds signaux numériques formés par des signaux EGIG :

grandeur de l'estomac,
grandeur du gros intestin,
grandeur de l'intestin grêle,
puissance de l'estomac,
puissance de l'intestin grêle,
puissance du gros intestin,
quotient grandeur/puissance de l'estomac,
quotient grandeur/puissance de l'intestin grêle,
quotient grandeur/puissance du gros intestin,
intégrale de la PSD dans la plage de 1 à 25 cycles par minute, CPM, puissance de plage complète,
quotient grandeur/puissance de plage complète (1 à 25 CPM),
fréquence de l'estomac,
fréquence de l'intestin grêle,
fréquence du gros intestin,
fréquence de plage complète (1 à 25 CPM),
coefficient de variation de la grandeur de l'estomac,
coefficient de variation du quotient grandeur/puissance de l'estomac,
coefficient de variation de la fréquence de l'intestin grêle,
coefficient de variation de la grandeur de l'intestin grêle,
coefficient de variation de la grandeur du gros intestin.

4. Système selon l'une quelconque des revendications 1 à 3, **caractérisé en ce que** le processeur est configuré pour déterminer un indicateur associé à une condition physiologique du patient, notamment un indicateur indicatif de stress psychologique ou d'anxiété ou un indicateur indicatif de maladie de reflux gastro-oesophagien, sur la base des données dérivées.

5. Système selon la revendication 4, **caractérisé en ce que** l'indicateur indicatif de stress psychologique ou d'anxiété est déterminé sur la base d'au moins une des données dérivées suivantes :

puissance totale normalisée de l'intestin grêle,
%pRR50,
racine carrée de la moyenne des différences au carré d'intervalles NN consécutifs, rMSSD, quotient du nombre de tous les intervalles RR détectés et du nombre d'occurrences de l'intervalle RR le plus fréquent, HRVi, coefficient de variation de l'intervalle RR, %CVRR, réponse galvanique cutanée normalisée, GSR normalisée.

Figure 1A

Figure 1B

Figure 2A

Figure 2B

Figure 2C

Figure 2D

*Figure 2E*

*Figure 3*

STAI 1
- Condition assessment
- Basic test battery
- ≈ 10 minutes

STAI 2
- Rest period
- ≈ 20 minutes

STAI 3
- 1st stress induction
- 2nd stress induction
- ≈ 24 minutes

STAI 4
- Rest period (after stress induction)
- ≈ 24 minutes

Figure 4

Figure 5

Figure 6

Figure 7

Error Bars: 95% CI

*Figure 8*

Error Bars: 95% CI

*Figure 9*

Error Bars: 95% CI

*Figure 10*

Error Bars: 95% CI

*Figure 11*

Error Bars: 95% CI

Figure 12

Figure 13

Figure 14

Figure 15

Figure 16

Figure 17

*Figure 18*

*Figure 19*

Figure 20

Figure 21

*Figure 22*

*Figure 23*

| Test | | Estimated | |
|---|---|---|---|
| | | reflux | |
| Observed | | not having reflux | having reflux |
| reflux | not having reflux | 7 | 4 |
| | having reflux | 1 | 9 |

Figure 24

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. BANACH et al.** Myoelectric activity of the stomach and esophageal pH changes in reflux disease. *Folia Med Cracov*, 2001, vol. 42 (1-2), 53-61
- **FRANCISCO MIGUEL VARGAS-LUNA et al.** Heart Rate Variability and Gastric Electrical Response to a Cold Pressor Task in Youth with Functional Dyspepsia. *Digestive Diseases and Sciences*, 2020, vol. 65 (4)
- **YUN LIU et al.** Psychological stress level detection based on electrodermal activity. *Behavioural Brain Research*, 2018, vol. 341
- The simultaneous recording and analysis both EGG and HRV signals. **S. PIETRASZEK et al.** PROCEEDINGS OF THE 31ST ANNUAL INTERNATIONAL CONFERENCE OF THE IEEE ENGINEERING IN MEDICINE AND BIOLOGY SOCIETY: ENGINEERING THE FUTURE OF BIOMED-ICINE, EMBC. IEEE, 2009, 396-399
- Inhibitory effects of stress on postprandial gastric myoelectrical activity and vagal tone in healthy subjects. **J. YIN et al.** NEUROGASTROENTEROLOGY AND MOTILITY. BLACKWELL SCIENTIFIC PUBLICATIONS, OXFORD, GB, 01 December 2004, vol. 16, 737-744
- **GÁBOR HÁMORI**. CHAID ALAPÚ DÖNTÉSI FÁK JELLEMZŐI. *Statisztikai Szemle*, 2001, vol. 79 (8)